(19) Europäisches Patentamt / European Patent Office / Office européen des brevets

(11) EP 4 595 946 A1

(12) **EUROPEAN PATENT APPLICATION**
published in accordance with Art. 153(4) EPC

(43) Date of publication:
**06.08.2025 Bulletin 2025/32**

(21) Application number: **23872058.5**

(22) Date of filing: **20.09.2023**

(51) International Patent Classification (IPC):
***A61K 8/891*** *(2006.01)* ***A61K 8/31*** *(2006.01)*
***A61K 8/895*** *(2006.01)* ***A61K 8/898*** *(2006.01)*
***A61Q 5/00*** *(2006.01)* ***A61Q 5/12*** *(2006.01)*

(52) Cooperative Patent Classification (CPC):
**A61K 8/31; A61K 8/891; A61K 8/894; A61K 8/895; A61K 8/898; A61Q 5/00; A61Q 5/10; A61Q 5/12; A61Q 19/00**

(86) International application number:
**PCT/JP2023/034004**

(87) International publication number:
**WO 2024/070830 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.09.2022 JP 2022157590**

(71) Applicant: **Kao Corporation**
**Chuo-ku,**
**Tokyo 103-8210 (JP)**

(72) Inventor: **MAEKAWA Tomoka**
**Tokyo 131-8501 (JP)**

(74) Representative: **Hoffmann Eitle**
**Patent- und Rechtsanwälte PartmbB**
**Arabellastraße 30**
**81925 München (DE)**

(54) **COSMETIC COMPOSITION**

(57) A cosmetic composition containing components (A) to (D) described below: (A) a film-forming polymer including an M unit represented by $(R^1)_3SiO_{1/2}$ (in which $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$; (B) a film-forming polymer other than the component (A); (C) a high-molecular-weight organopolysiloxane; and (D) an organopolysiloxane other than the components (A) to (C), the organopolysiloxane having a polyalkylene oxide moiety and a cationic group.

## Description

Technical Field

The present invention relates to a cosmetic composition.

Background Art

It is known that hair, which is a type of keratin substance, is damaged by external factors in daily life, chemical treatments such as perming or coloring, and the like, and becomes difficult to handle. To solve such a situation, a conditioning agent or a styling agent is used to impart a gloss or a smooth feeling to the hair. However, many of these effects are lost by one time of shampooing or by a physical force received in daily life, such as friction between the hair and a pillow or the like during sleeping. Thus, there is a demand for a product which can sustain these effects.

Accordingly, in recent years, in the field of cosmetics, a technique for forming a hydrophobic film has been studied as a method for imparting various performances such as a gloss and a good feeling to a keratin substance such as skin or hair.

A technique using a silicone-based film-forming polymer is known as a hydrophobic film-forming polymer. For example, JP 2016-503433 T (Patent Literature 1) discloses a composition containing a silicone resin and a silicone rubber, in which a mixture of the silicone resin and the silicone rubber has a softening point of higher than 50°C and an elastic modulus of $10^6$ Pa or less at ambient temperature, and discloses that when a formulation containing the composition is applied to hair, it gives a smooth feeling and a strong gloss.

One of known hair dyeing techniques is a temporary hair dyeing technique in which a film containing a coloring agent such as a pigment is formed on the surface of hair to dye the hair. Temporary hair dyes are widely accepted because coloring can be easily performed with little damage to hair, but the color is likely to be lost by shampooing, thereby leaving room for improvement in washing durability. Thus, studies have been conducted on a technique for easily performing coloring and improving sustainability of the coloring effect.

For example, JP 2021-187850 A (Patent Literature 2) discloses that when a cosmetic composition containing a silicone film-forming agent, a high-molecular-weight organopolysiloxane, and an organopolysiloxane having a polyalkylene oxide moiety and a cationic group is applied to a keratin substance such as skin or hair, it is possible to impart a good feeling in both a dry state and a wet state, durability against friction is excellent, and sustainability of these effects is excellent even after washing.

Meanwhile, it is known that sebum secreted from sebaceous glands always spreads in the form of a thin film on the surface of skin and hair and plays a role in physically or chemically protecting the skin and the hair. In addition, oily components such as silicone contained in skin or hair cosmetics also remain on the surface of the skin and the hair. Thus, a cosmetic composition is required to exhibit a desired performance even in the presence of an oily component.

JP H07-196449 A (Patent Literature 3) discloses, as an eye makeup cosmetic capable of forming a uniform film and having good water resistance and sebum resistance, an eye makeup cosmetic characterized by containing trimethylsiloxysilicate, an acrylic-silicone-based graft copolymer produced by radical copolymerization of a dimethylpolysiloxane compound having a radical polymerizable group at one terminal of the molecular chain and a radical polymerizable monomer mainly composed of acrylate and/or methacrylate, and a low-boiling-point silicone oil and/or a low-boiling-point isoparaffin-based hydrocarbon oil.

Summary of Invention

The present invention relates to the following.

[1] A cosmetic composition containing components (A) to (D) described below:

(A) a film-forming polymer including an M unit represented by $(R^1)_3SiO_{1/2}$ (in which $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$;
(B) a film-forming polymer other than the component (A);
(C) a high-molecular-weight organopolysiloxane; and
(D) an organopolysiloxane other than the components (A) to (C), the organopolysiloxane having a polyalkylene oxide moiety and a cationic group.

[2] A method for treating a keratin substance, the method including applying the composition described in [1] above to a keratin substance and then drying the composition.

Description of Embodiments

Patent Literatures 1 and 2 do not mention the performance expression in a case where the composition is applied to a target in which an oily component such as sebum is present.

Patent Literature 3 mentions the sebum resistance of the eye makeup cosmetic, but the sebum resistance evaluated in Examples is an evaluation of the scratch resistance of the cosmetic when artificial sebum is applied after application of the cosmetic. Thus, Patent Literature 3 also does not mention the performance in the case where the cosmetic composition is applied to a target in which sebum is present.

The present invention relates to providing a cosmetic composition which, when applied to a target in which an oily component such as sebum is present, can impart a good feeling to the target and can form a film having excellent washing durability.

The present inventor has found that the above-described problems can be solved by a cosmetic composition containing a silicone-based film-forming polymer having a specific constituent unit and another film-forming polymer, a high-molecular-weight organopolysiloxane, and an organopolysiloxane having a polyalkylene oxide moiety and a cationic group, and has completed the present invention.

When the cosmetic composition of the present invention is applied to a target in which an oily component such as sebum is present, a good feeling can be imparted to the target, and a film having excellent washing durability can be formed.

[Definitions]

The term "polymer" as used herein means a compound corresponding to repetition of one unit or a plurality of units (such a unit is derived from a compound known as a monomer). This unit or these units are repeated at least two times, preferably at least three times.

The term "hair" as used herein primarily means head hair.

The term "hydrophobic" as used herein means that a substance has a solubility in water of less than 1 mass% at 25°C.

The term "film-forming" as used herein means leaving a film on a base material to which it is applied.

The term "washing durability" as used herein primarily means that an effect imparted by a cosmetic composition is sustained even when washing is performed after application of the composition to a target, and primarily means that color loss due to shampooing is less likely to occur in a case where the composition is a hair dye.

The term "volatile" as used herein means having a boiling point of 260°C or lower at normal pressure.

[Cosmetic Composition]

The cosmetic composition of the present invention contains components (A) to (D) described below:

(A) a film-forming polymer including an M unit represented by $(R^1)_3SiO_{1/2}$ (in which $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$;
(B) a film-forming polymer other than the component (A);
(C) a high-molecular-weight organopolysiloxane; and
(D) an organopolysiloxane other than the components (A) to (C), the organopolysiloxane having a polyalkylene oxide moiety and a cationic group.

Hereinafter, "the M unit represented by $(R^1)_3SiO_{1/2}$ and the Q unit represented by $SiO_{4/2}$" are also referred to as an "MQ unit". In the following description, the cosmetic composition may be referred to simply as a "composition".

Since the cosmetic composition of the present invention has the above-described configuration, in a case where the cosmetic composition is applied to a target in which an oily component such as sebum is present, it is possible to impart a good feeling to the target and form a film having excellent washing durability.

The reason why the composition of the present invention exhibits the above-described effects is not clear, but is presumed as follows.

The composition of the present invention containing the component (A) and the component (B), which are hydrophobic film-forming agents, can form a hydrophobic film when applied to a target.

The component (A) contains a rigid MQ unit, and thus a hard film can be formed. The component (A) is also advantageous in that it is unlikely to be plasticized by an oily component such as sebum. Meanwhile, the component (A) has low toughness, and thus the formed film tends to be brittle, and is difficult to impart a good feeling to the target.

The component (B) usually has higher toughness than that of the component (A), and thus it is considered that the

flexibility of the formed film can be enhanced by using the component (B) in the composition of the present invention. As a result, it is considered that the scratch resistance of the film is improved to enhance the washing durability, and further, the effect of improving the feeling of the treated target is also achieved. In a case where a functional powder described below is blended in the composition, the functional powder can be held in the film to enhance various functions and their sustainability.

It is considered that the component (C) and the component (D) exhibit an effect of accelerating the phase separation of the components in the formed hydrophobic film, whereby the formed film is hardly affected by plasticization or the like even in the presence of an oily component such as sebum, and can exhibit a desired effect. More specifically, in a case where the component (C) having high hydrophobicity and low surface tension is phase-separated in the formed hydrophobic film, it is considered that a large amount of the component (C) is present on the film surface side (air side). Meanwhile, the component (D) exhibits the effect of accelerating the phase separation in the hydrophobic film, and the component (D) itself is present in a large amount on the target side in the film. It is considered that the component (A) and the component (B) are unevenly distributed between the component (C) layer and the component (D) layer. The expression "phase-separated structure" as used herein is not limited to a structure having a clear interface at the boundary of layers.

According to the composition of the present invention, it is considered that when the film having the phase-separated structure is formed, the composition is hardly affected by an oily component such as sebum and can effectively exhibit the effect of improving a feeling for a target and the effect of improving washing durability. In addition, when the component (C) has a high molecular weight, it is considered that the above-described phase-separated structure is easily formed, and in addition, the occurrence of stickiness of the film is suppressed, and a good feeling is easily imparted.

The mechanism of action of the present invention is not limited to the above-described one.

<Application Target of Composition>

A target to which the cosmetic composition of the present invention is applied is preferably a keratin substance.

Examples of the keratin substance include keratin substances such as skin, hair, eyelashes, eyebrows, and nails.

<Form of Composition>

The composition of the present invention is preferably a cosmetic composition for the keratin substance, more preferably one or more selected from the group consisting of a skin cosmetic composition and a hair cosmetic composition, and even more preferably a hair cosmetic composition.

Examples of the product form of the skin cosmetic composition include a body soap, a facial wash, a bath agent, a deodorant preparation, a makeup cosmetic, a sunscreen, a makeup base, a milky lotion, a beauty essence, and a cream.

Examples of the product form of the hair cosmetic composition include a shampoo, a rinse, a conditioner, a treatment agent (including a non-rinsed type), a styling agent, a hair dye, a hair growth tonic, and a permanent agent. Among them, from the viewpoint of effectiveness of the effect of the present invention, a conditioner, a treatment agent, a styling agent, a hair dye, or a hair growth tonic is preferable, and a hair dye is more preferable.

The composition of the present invention is preferably a composition which is used without being rinsed off after being applied to a keratin substance, from the viewpoint of the effect of the present invention that in a case where the composition is applied to a target in which an oily component such as sebum is present, it is possible to impart a good feeling and form a film having excellent washing durability. For example, in a case where the composition of the present invention is a hair dye, the hair dye is preferably a temporary hair dye which is applied to hair to form a colored film and is used without being rinsed off.

Hereinafter, the respective components contained in the composition of the present invention will be described.

<Component (A): Film-Forming Polymer Containing M Unit and Q Unit>

The composition of the present invention contains, as the component (A), a film-forming polymer including an M unit represented by $(R^1)_3SiO_{1/2}$ and a Q unit represented by $SiO_{4/2}$ (in which $R^1$ represents a hydrocarbon group having 1 or more and 12 or less carbons and being optionally substituted with fluorine, or a hydroxy group, and a plurality of $R^1$ may be the same or different).

The composition of the present invention contains the component (A), and thus, when applied to a target, the composition can form a hard hydrophobic film which is hardly plasticized even when it comes into contact with an oily component. In a case where a functional powder described below is blended in the composition, the functional powder can be held in the film to enhance various functions and their sustainability.

In the M unit, $R^1$ is a hydrocarbon group having 1 or more and 12 or less carbons and being optionally substituted with fluorine, or a hydroxy group. The number of carbons of the hydrocarbon group is 1 or more, and is preferably 9 or less, more preferably 6 or less, and even more preferably 4 or less, from the viewpoint of improving film formability and washing durability.

The hydrocarbon group may be either an aliphatic group or an aromatic group, and examples thereof include an alkyl group, an alkenyl group, an aryl group, and an aralkyl group. The alkyl group and the alkenyl group may be linear or branched.

Among them, from the viewpoint of availability and stability, the hydrocarbon group is preferably an alkyl group, an aryl group, or an aralkyl group.

Examples of the alkyl group include a methyl group, an ethyl group, an n-propyl group, an isopropyl group, various butyl groups, various pentyl groups, various hexyl groups, various heptyl groups, various octyl groups, various nonyl groups, various decyl groups, various undecyl groups, and various dodecyl groups. The term "various" means a linear or branched hydrocarbon group. For example, the term "various butyl groups" includes "an n-butyl group, a sec-butyl group, an isobutyl group, and a tert-butyl group".

Examples of the aryl group include a phenyl group, a toluyl group, a dimethylphenyl group, and a naphthyl group, and a phenyl group is preferable.

Examples of the aralkyl group include a benzyl group, a phenylethyl group, a phenylpropyl group, and a phenylbutyl group, and a phenylpropyl group is preferable.

In a case where $R^1$ is substituted with fluorine, at least one hydrogen atom in the hydrocarbon group need only be substituted with a fluorine atom.

$R^1$ is preferably an alkyl group having 1 or more and 12 or less carbons and being optionally substituted with fluorine, an aryl group having 6 or more and 12 or less carbons, or an aralkyl group having 7 or more and 12 or less carbons, more preferably an alkyl group having 1 or more and 8 or less carbons and being optionally substituted with fluorine, or a phenyl group, and even more preferably an alkyl group having 1 or more and 6 or less carbons and being optionally substituted with fluorine, or a phenyl group, from the viewpoint of improving film formability and washing durability. The fluorine-substituted alkyl group is preferably a group represented by $CF_3$-R- (in which R is an alkylene group having 2 or more and 7 or less carbons, preferably 2 or more and 5 or less carbons).

$R^1$ is more preferably a trifluoropropyl group, an alkyl group having 1 or more and 4 or less carbons, or a phenyl group, even more preferably a trifluoropropyl group, a methyl group, an ethyl group, an n-propyl group, an isopropyl group, or an n-butyl group, still more preferably a trifluoropropyl group, a methyl group, or an n-propyl group, and still even more preferably a methyl group.

The component (A) need only be any film-forming polymer having the above-described MQ unit, and is preferably a hydrophobic film-forming polymer having the MQ unit.

The component (A) may further contain a D unit represented by $(R^1)_2SiO_{2/2}$ (in which $R^1$ is the same as described above), from the viewpoint of improving film formability and washing durability.

However, from the viewpoint of obtaining a synergistic effect by using the component (A) and the component (B), it is preferable that the component (A) is substantially free of a T unit represented by $R^1SiO_{3/2}$ (in which $R^1$ is the same as described above). The expression "substantially free of" means that the constituent proportion in the silicone resin is less than 1 mol%.

From the viewpoint of improving film formability and washing durability, the component (A) is preferably a silicone resin represented by an average formula $(R^1)_mSiO_{(4-m)/2}$ (in which $R^1$ represents a hydrocarbon group having 1 or more and 12 or less carbons and being optionally substituted with fluorine, or a hydroxy group, a plurality of $R^1$ may be the same or different, and m is an average number of more than 0 and less than 3), and including an M unit represented by $(R^1)_3SiO_{1/2}$ and a Q unit represented by $SiO_{4/2}$.

The silicone resin represented by the average formula and including the Q unit has a crosslinked structure in the molecule. It is considered that with this structure, a film having higher washing durability can be formed even in the presence of an oily component such as sebum. The silicone resin does not include a cured polyorganosiloxane powder which is infusible, has no softening point, and is generally insoluble in an organic solvent.

The component (A) is more preferably a silicone resin represented by $[SiO_{4/2}]_c[(R^1)_3SiO_{1/2}]_d$ (in which c and d are numbers of average repeating units, and c > 0 and d > 0 are satisfied), from the viewpoint of improving film formability and washing durability.

Examples of the component (A) include trimethylsiloxysilicate, phenylpropyldimethylsiloxysilicate, fluorine-modified alkyldimethylsiloxysilicate, and crosspolymers produced by crosslinking these siloxysilicates with dimethiconol or the like, and one or two or more of these can be used. Examples of the fluorine-modified alkyldimethylsiloxysilicate include trifluoroalkyldimethyltrimethylsiloxysilicate, and trifluoropropyldimethyltrimethylsiloxysilicate having the INCI name "trifluoropropyldimethyl/trimethylsiloxysilicate". Examples of the crosspolymers produced by crosslinking siloxysilicates with dimethiconol or the like include the INCI name "(trimethylsiloxysilicate/dimethiconol) crosspolymer".

Among them, from the viewpoint of improving film formability and washing durability, the component (A) is preferably one or more selected from the group consisting of trimethylsiloxysilicate, trifluoropropyldimethyltrimethylsiloxysilicate, and (trimethylsiloxysilicate/dimethiconol) crosspolymer, more preferably one or more selected from the group consisting of trimethylsiloxysilicate and trifluoropropyldimethyltrimethylsiloxysilicate, and even more preferably trimethylsiloxysilicate.

In the component (A), examples of commercially available products of trimethylsiloxysilicate include KF-7312J (50 mass% decamethylcyclopentasiloxane solution), KF-9021 (50 mass% decamethylcyclopentasiloxane solution), X-21-5249 (50 mass% decamethylcyclopentasiloxane solution), X-21-5595 (60 mass% isododecane solution), and X-21-5616 (60 mass% isododecane solution) (all available from Shin-Etsu Chemical Co., Ltd.), SS4267 (35 mass% dimethylpolysiloxane solution), SR1000, SS4230 (45 mass% cyclopentasiloxane solution), SS4267 (35 mass% dimethylpolysiloxane solution), Silsoft74 (75 mass% isododecane solution) (all available from Momentive Performance Materials Inc.), BY11-018 (30 mass% cyclopentasiloxane solution), MQ-1600 Solid Resin (all available from Dow Toray Co., Ltd.), and BELSIL TMS 803 (available from Wacker Asahikasei Silicone Co., Ltd.).
Examples of commercially available products of phenylpropyldimethylsiloxysilicate include SilShine 151 (available from Momentive Performance Materials Inc.).
Examples of commercially available products of the fluorine-modified alkyldimethylsiloxysilicate include XS66-B8226 (50 mass% cyclopentasiloxane solution), XS66-C1191, and XS66-B8636 (50 mass% dimethicone solution) (all available from Momentive Performance Materials Inc.) having the INCI name "trifluoropropyldimethyl/trimethylsiloxysilicate".
Examples of commercially available products of the trimethylsiloxysilicate crosspolymer include DOWSIL FC-5002 IDD Resin Gum (40 mass% isododecane solution of (trimethylsiloxysilicate/dimethiconol) crosspolymer) (available from Dow Toray Co., Ltd.).

<Component (B): Film-Forming Polymer other than Component (A)>

The composition of the present invention contains, as the component (B), a film-forming polymer other than the component (A). It is considered that the use of the component (B) can improve the feeling and washing durability of a target in which an oily component is present, as compared with a case where the component (A) is used alone.
The component (B) is preferably a hydrophobic film-forming polymer from the viewpoint of compatibility with the component (A) and from the viewpoint of being capable of imparting good feeling to a target in which an oily component is present and forming a film having excellent washing durability.
The hydrophobic film-forming polymer to be used can be either a silicone-based polymer or a non-silicone-based polymer, and these polymers may be used in combination. The silicone-based polymer according to the component (B) refers to a film-forming polymer other than the component (A) including a silicone structure, and the non-silicone-based polymer refers to a film-forming polymer including no silicone structure.
From the viewpoint of compatibility with the component (A) and the viewpoint of being capable of imparting a good feeling to a target in which an oily component is present and forming a film having excellent washing durability, the component (B) preferably contains a silicone-based polymer. The content of the silicone-based polymer in the component (B) is preferably 50 mass% or more, more preferably 70 mass% or more, even more preferably 80 mass% or more, and still more preferably 90 mass% or more, and is 100 mass% or less.
Examples of the non-silicone-based film-forming polymer to be used as the component (B) include a non-silicone-based cationic polymer, a non-silicone-based anionic polymer, a non-silicone-based nonionic polymer, and a non-silicone-based amphoteric polymer.
Examples of the cationic polymer include vinylpyrrolidone-N,N-dimethylaminoethyl methacrylate copolymer diethyl sulfate (polyquaternium-11) (GAFQUAT 440, GAFQUAT 734 (available from IPS Japan Ltd.), H.C. polymer (available from Osaka Organic Chemical Industry Ltd.)), vinylpyrrolidone-dimethylaminopropylmethacrylamide-methacrylamidopropyllauryldimonium chloride (polyquaternium-55) (STYLESE W-20 (available from ISP Japan Ltd.)), polydimethylmethylenepiperidinium chloride (polyquaternium-6) (Merquat 100 (available from The Lubrizol Corporation)), dimethyldiallylammonium chloride-acrylamide copolymer (polyquaternium-7) (Merquat 550 (available from The Lubrizol Corporation)), ammonium-modified hydroxyethylcellulose (SoftCAT SL-30 polymer (available from The Dow Chemical Company)), N,N-dimethylaminoethyl methacrylate diethyl sulfate-N, N-dimethylacrylamide-polyethylene glycol dimethacrylate copolymer (polyquaternium-52), vinylpyrrolidone-alkylamino(meth)acrylate copolymer, vinylpyrrolidone-alkylamino(meth)acrylate-vinylcaprolactam copolymer, alkylacrylamide-(meth)acrylate-alkylaminoalkylacrylamide-polyethylene glycol (meth)acrylate copolymer, adipic acid-dimethylaminohydroxypropylethylenetriamine copolymer, and acrylamide-acrylic acid-DMAPA-methacrylic acid-methoxy PEG copolymer.
Examples of the anionic polymer include (acrylates/diacetoneacrylamide) copolymers (Plussize L-9540B, Plussize L-9600U, Plussize L-53, and the like (available from Goo Chemical Co., Ltd.)), (acrylates/alkyl acrylate (C1-18)/alkyl (C1-8) acrylamide) copolymers (Plussize L-9909B, Plussize L-9900 (available from Goo Chemical Co., Ltd.)), an alkyl acrylate-octylacrylamide copolymer (Dermacryl 79 (available from AkzoNobel)), a vinyl acetate-crotonic acid-vinyl neodecanoate copolymer (RESYN 28-2930 (available from AkzoNobel)), acrylic acid-acrylamide-ethyl acrylate copolymers (Ultrahold 8, Ultrahold Strong (available from BASF)), alkyl acrylate copolymers (Aniset NF-1000, Aniset HS-300, and the like (available from Osaka Organic Chemical Industry Ltd.)), and an isophorone diisocyanate-dimethylolpropionic acid-(polyoxyethylene-polyoxypropylene)4,4'-isopropylidenediphenol copolymer (DynamX (available from

AkzoNobel)).

Examples of the nonionic polymer include an (acrylates/methoxymethacrylate PEG-23) copolymer (Plussize L-188K (available from Goo Chemical Co., Ltd.)), (dimethylacrylamide/hydroxyethylacrylate/methoxyethylacrylate) copolymers (Plussize L-2700, Plussize L-2714 (available from Goo Chemical Co., Ltd.)), a (hydroxyethylacrylate/methoxyethylacrylate) copolymer (Plussize L-2200 (available from Goo Chemical Co., Ltd.)), polyvinylpyrrolidones (Luviskol K17, Luviskol K30, Luviskol K90 (available from BASF)), vinylpyrrolidone-vinyl acetate copolymers (Luviskol VA73E, Luviskol 37E (available from BASF)), vinyl methyl ether-alkyl maleate copolymers (GANTREZ A-425, GANTREZ ES-225 (available from Ashland Specialty Ingredients)), a vinylpyrrolidone-methacrylamide-vinylimidazole copolymer (Lubriset Clear (available from BASF)), and polyvinylcaprolactam (Luviskol Plus (available from BASF)).

Examples of the amphoteric polymer include an acrylates-lauryl acrylate-stearyl acrylate-ethylamine oxide methacrylate copolymer (DIAFORMER Z651 (available from Mitsubishi Chemical Corporation)), methacryloyloxyethylcarboxybetaine-alkyl methacrylate copolymers (YUKAFORMER M75, YUKAFORMER R205 (available from Mitsubishi Chemical Corporation)), an octylacrylamide-hydroxypropyl acrylate-butylaminoethyl methacrylate copolymer (AMPHOMER 28-4910 (available from AkzoNobel)), and an octylacrylamide-hydroxypropyl acrylate-butylaminoethyl methacrylate copolymer (AMPHOMER SH30 (available from AkzoNobel)).

Examples of the silicone polymer to be used as the component (B) include one or more selected from the group consisting of the following components (B1) to (B6):

(B1) a silicone resin containing a T unit represented by $R^1SiO_{3/2}$ and being substantially free of a Q unit represented by $SiO_{4/2}$ (in which $R^1$ is as described above);
(B2) an acrylic silicone polymer;
(B3) a silicone-modified alicyclic structure-containing polymer;
(B4) silicone-modified pullulan;
(B5) polyurea/urethane silicone; and
(B6) oxazoline-modified silicone.

(Component (B1): Silicone Resin)

The component (B1) is a silicone resin containing a T unit represented by $R^1SiO_{3/2}$ and being substantially free of a Q unit represented by $SiO_{4/2}$ (in which $R^1$ is as described above).

The component (B1) preferably further contains one or more units selected from the group consisting of an M unit represented by $(R^1)_3SiO_{1/2}$, and a D unit represented by $(R^1)_2SiO_{2/2}$. $R^1$ is as described above.

From the viewpoint of imparting a good feeling to a target in which an oily component is present and forming a film having excellent washing durability, the component (B1) is preferably a silicone resin that is represented by $[R^1SiO_{3/2}]_a[(R^1)_3SiO_{1/2}]_b$ (in which a and b are the average number of repeating units and $a > 0$ and $b \geq 0$ are satisfied), and includes a T unit, and may include an M unit. The expression "substantially free of" means that the constituent proportion in the silicone resin is less than 1 mol%.

$R^1$ is as described above, and is preferably an alkyl group having 1 or more and 4 or less carbons or a phenyl group, more preferably a methyl group, an ethyl group, an n-propyl group, or an isopropyl group, and even more preferably a methyl group, an n-propyl group, or an isopropyl group.

Examples of the component (B1) include polysilsesquioxanes such as polymethylsilsesquioxane, polypropylsilsesquioxane, polyphenylsilsesquioxane, polymethylphenylsilsesquioxane, and fluorine-modified alkyldimethylpolysilsesquioxane, and one or two or more of these may be used. Examples of the fluorine-modified alkyldimethylpolysilsesquioxane include an INCI name "(trifluoropropyldimethylsiloxy/trimethylsiloxy) silsesquioxane".

Among them, from the viewpoint of imparting a good feeling to a target in which an oily component is present and forming a film having excellent washing durability, the component (B1) is preferably one or more selected from the group consisting of polymethylsilsesquioxane and polypropylsilsesquioxane, and is more preferably polymethylsilsesquioxane.

Examples of commercially available products of the component (B1) include SilForm Flexible Resin (polymethylsilsesquioxane), SilForm FR-5 (polydimethylsiloxane solution of (trifluoropropyldimethylsiloxy/trimethylsiloxy) silsesquioxane) (available from Momentive Performance Materials, Inc.), DOWSIL 680 ID Fluid (75 mass% isododecane solution of polypropylsilsesquioxane) (available from Dow Toray Co., Ltd.), SR-21 (polyphenylsilsesquioxane), SR-23 (polyphenylsilsesquioxane), and SR-33 (polymethylphenylsilsesquioxane) (available from Konishi Chemical Ind. Co., Ltd.).

(Component (B2): Acrylic Silicone Polymer)

Examples of the component (B2) include acrylic polymers having a carbosiloxane dendrimer structure in a side chain, acrylic-silicone-based graft copolymers, and graft copolymers or alternating block copolymers in which a constituent unit including a polysiloxane group and a constituent unit including a polymer of an unsaturated monomer are bonded via a sulfide bond.

Examples of the acrylic polymer having a carbosiloxane dendrimer structure in a side chain include a silicone dendrimer-acrylic copolymer, which can be produced in accordance with a production method described in, for example, JP H11-1530 A or JP 2000-63225 A.

The acrylic polymer having a carbosiloxane dendrimer structure in a side chain is preferably an INCI name "(acrylates/polytrimethylsiloxymethacrylate) copolymer".

Examples of commercially available products of the (acrylates/polytrimethylsiloxymethacrylate) copolymer include DOWSIL FA 4001 CM Silicone Acrylate (30 mass% decamethylcyclopentasiloxane solution), DOWSIL FA 4002 ID Silicone Acrylate (40 mass% isododecane solution), DOWSIL FA 4003 DM Silicone Acrylate (40 mass% dimethicone solution), and DOWSIL FA 4004 ID Silicone Acrylate (40 mass% isododecane solution) (all available from Dow Toray Co., Ltd.).

Examples of the acrylic-silicone-based graft copolymer include a radical polymer of an organopolysiloxane compound having a radical polymerizable group at one terminal of the molecular chain and a radical polymerizable monomer mainly composed of acrylate and/or methacrylate.

The radical polymer of an organopolysiloxane compound having a radical polymerizable group at one terminal of the molecular chain and a radical polymerizable monomer mainly composed of acrylate and/or methacrylate can be, for example, those described in JP H02-25411 A and JP H02-132141 A and acrylic-silicone-based graft copolymers described in JP H03-162442 A and JP 2003-104825 A.

The acrylic-silicone-based graft copolymer is preferably an INCI name "(acrylates/dimethicone) copolymer". Examples of commercially available products thereof include KP-545 (30 mass% decamethylcyclopentasiloxane solution), KP-549 (40 mass% methyltrimethicone solution), and KP-550 (40 mass% isododecane solution) (all available from Shin-Etsu Chemical Co., Ltd.).

Examples of the graft copolymer or alternating block copolymer in which a constituent unit including a polysiloxane group and a constituent unit including a polymer of an unsaturated monomer are bonded via a sulfide bond include a graft copolymer or alternating block copolymer described in JP H06-92825 A.

Among them, from the viewpoint of imparting a good feeling to a target in which an oily component is present and forming a film having excellent washing durability, the component (B2) is preferably one or more selected from the group consisting of acrylic polymers having a carbosiloxane dendrimer structure in a side chain and acrylic-silicone-based graft copolymers, is more preferably one or more selected from the group consisting of (acrylates/polytrimethylsiloxymethacrylate) copolymers and (acrylates/dimethicone) copolymers, even more preferably contains an (acrylates/polytrimethylsiloxymethacrylate) copolymer, and is still even more preferably an (acrylates/polytrimethylsiloxymethacrylate) copolymer.

(Component (B3): Silicone-Modified Alicyclic Structure-Containing Polymer)

Examples of the silicone-modified alicyclic structure-containing polymer as the component (B3) include silicone-modified cyclic polyolefins, and preferable examples thereof include silicone-modified polynorbornenes represented by General Formula (B3-1) described below.

[Chem. 1]

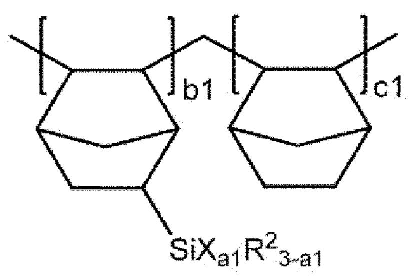

(B3-1)

(In the formula, $R^2$ each independently represents an alkyl group having 1 or more and 12 or less carbons, and X is a group represented by Formula (i) described below, a1 is an integer of 1 or more and 3 or less, and b1 and c1 represent the number of repeating units, and are each independently an integer of 1 or more.)

[Chem. 2]

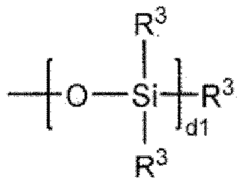 (i)

(In the formula, $R^3$ each independently represents a hydrocarbon group having 1 or more and 12 or less carbons, and d1 is an integer of 1 or more and 5 or less.)

In General Formula (B3-1), $R^2$ is preferably a methyl group, an ethyl group, an n-propyl group, a butyl group, or a pentyl group, and is more preferably a methyl group.

X is a group represented by Formula (i), and in Formula (i), $R^3$ each independently represents a hydrocarbon group having 1 or more and 12 or less carbons. $R^3$ is preferably an alkyl group having 1 or more and 12 or less carbons or a phenyl group, more preferably an alkyl group having 1 or more and 3 or less carbons, and even more preferably a methyl group. d1 is an integer of 1 or more and 5 or less, and d1 = 1 is preferably satisfied from the viewpoint of versatility. That is, X is preferably a trimethylsiloxy group.

a1 is an integer of 1 or more and 3 or less. For example, the polymer may include a repeating unit of a1 = 2 and a repeating unit of a1 = 3 in a mixed manner. From the viewpoint of versatility, a1 is preferably 3.

The ratio of b1 to c1 in General Formula (B3-1) is preferably b1/c1 = 20/80 or more and 90/10 or less (mol/mol), more preferably 30/70 or more and 80/20 or less (mol/mol), and even more preferably 50/50 or more and 70/30 or less (mol/mol). The ratio of b1 to c1 can be determined by $^1$H-NMR measurement.

The silicone-modified polynorbornene is more preferably a silicone-modified polynorbornene represented by Formula (B3-2) described below:

[Chem. 3]

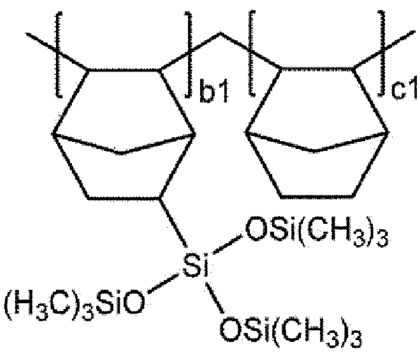 (B3-2)

(In the formula, b1 and c1 are as described above.)

Examples of the silicone-modified polynorbornene represented by Formula (B3-2) include a compound represented by the INCI name "(norbornene/tris(trimethylsiloxy)silylnorbornene) copolymer".

Examples of commercially available silicone-modified polynorbornene include NBN-30-ID (isododecane solution of (norbornene/tris(trimethylsiloxy)silylnorbornene) copolymer) (available from Shin-Etsu Chemical Co., Ltd.).

(Component (B4): Silicone-Modified Pullulan)

Examples of the component (B4) include pullulan having a silicone structure in a side chain thereof. Specifically, preferred is silicone-modified pullulan in which at least some of hydrogen atoms of OH groups in pullulan are substituted with a group represented by General Formula (ii) described below.
[Chem. 4]

$$-R^4\text{-}SiX_{a1}R^2_{3\text{-}a1} \qquad \text{(ii)}$$

In the formula, $R^4$ is a single bond or a divalent organic group. $R^2$, X, and a1 are as described above, and from the viewpoint of versatility, X is preferably a trimethylsiloxy group, and a1 is preferably 3.

In General Formula (ii), $R^4$ is preferably a divalent organic group, more preferably a divalent group represented by

General Formula (iii) or (iv) described below, and even more preferably a divalent group represented by General Formula (iv) described below.

[Chem. 5]

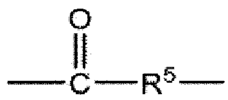

(iii)

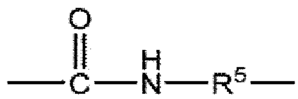

(iv)

In the formula, $R^5$ is an alkylene group having 1 or more and 10 or less carbons, and examples thereof include a methylene group, an ethylene group, a trimethylene group, a propylene group, and a butylene group. Among them, an ethylene group, a trimethylene group, and a propylene group are preferable, and a trimethylene group or a propylene group is more preferable.

Examples of commercially available silicone-modified pullulan include TSPL-30-ID (isododecane solution of tri(trimethylsiloxy) silylpropyl carbamate pullulan), and TSPL-30-D5 (cyclopentasiloxane solution of tri(trimethylsiloxy) silylpropyl carbamate pullulan) (all available from Shin-Etsu Chemical Co., Ltd.).

(Component (B5): Polyurea/Urethane Silicone)

Examples of the component (B5) include a polysiloxane/polyurea/polyurethane block terpolymer. For example, a dimethylpolysiloxane/urea copolymer with an INCI name "polyureadimethicone" is exemplified.

The polymer can be produced by copolymerization of an $\alpha,\omega$-aminosilicone and a diisocyanate. Examples of commercially available polyurea/urethane silicone include "Wacker-Belsil UD 60", "Wacker-Belsil UD 80", "Wacker-Belsil UD 140", and "Wacker-Belsil UD 200" (all available from Wacker Chemie AG).

(Component (B6): Oxazoline-Modified Silicone)

Examples of the oxazoline-modified silicone as the component (B6) include organopolysiloxane formed by bonding a poly(N-acylalkyleneimine) segment composed of a repeating unit represented by General Formula (B6-1) described below:

[Chem. 6]

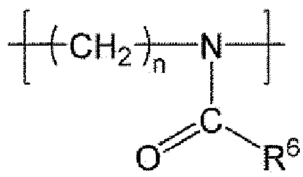

(B6-1)

(wherein $R^6$ represents a hydrogen atom, an alkyl group having 1 or more and 22 or less carbons, an aralkyl group having 7 or more and 22 or less carbons, or an aryl group having 6 or more and 22 or less carbons, and n is 2 or 3)
via an alkylene group containing a heteroatom to at least two of silicon atoms of an organopolysiloxane segment constituting a main chain.

Although at least two poly(N-acylalkyleneimine) segments can be bonded to any silicon atom constituting the organopolysiloxane segment via an alkylene group containing a heteroatom, the poly(N-acylalkyleneimine) segments are preferably bonded to one or more silicon atoms excluding both terminals via the alkylene group, and are more preferably bonded to two or more silicon atoms excluding both terminals via the alkylene group.

The alkylene group containing a heteroatom functions as a linking group for the poly(N-acylalkyleneimine) segments. Examples of such an alkylene group include an alkylene group having 2 or more and 20 or less carbons and containing 1 to 3 nitrogen atoms, oxygen atoms, or sulfur atoms.

In $R^6$ in General Formula (B6-1), the alkyl group having 1 or more and 22 or less carbons is, for example, a linear, branched, or cyclic alkyl group having 1 or more and 22 or less carbons. Specific examples thereof include a methyl group, an ethyl group, an n-propyl group, an isopropyl group, an n-butyl group, a tert-butyl group, a pentyl group, a hexyl group, a

cyclohexyl group, a heptyl group, an octyl group, a nonyl group, a decyl group, an undecyl group, a dodecyl group, an octadecyl group, a nonadecyl group, an eicosyl group, and a docosyl group. Among these, an alkyl group having 1 or more and 10 or less carbons is preferable, and an alkyl group having 1 or more and 6 or less carbons is more preferable.

In $R^6$ in General Formula (B6-1), specific examples of the aralkyl having 7 or more and 22 or less carbons include a benzyl group, a phenethyl group, a trityl group, a naphthylmethyl group, and an anthracenylmethyl group. Among these, an aralkyl group having 7 or more and 14 or less carbon atoms is preferable, and an aralkyl group having 7 or more and 10 or less carbon atoms is more preferable.

In $R^6$ in General Formula (B6-1), specific examples of the aryl group having 6 or more and 22 or less carbons include a phenyl group, a tolyl group, a xylyl group, a naphthyl group, a biphenyl group, an anthryl group, and a phenanthryl group. Among these, an aryl group having 6 or more and 14 or less carbons is preferable, and an aryl group having 6 or more and 12 or less carbons is more preferable.

Among the above, $R^6$ in General Formula (B6-1) is preferably an alkyl group having 1 or more and 22 or less carbons, more preferably 1 or more and 10 or less carbons, even more preferably 1 or more and 6 or less carbons, and still more preferably 1 or more and 3 or less carbons.

Examples of the oxazoline-modified silicone as the component (B6) include poly(N-formylethyleneimine) organosiloxane, poly(N-acetylethyleneimine)organosiloxane, and poly(N-propionylethyleneimine)organosiloxane, and one or two or more thereof can be used. Among them, the polymer represented by an INCI name "polysilicone-9" is preferably used.

One type or two or more types can be used as the component (B).

From the viewpoint of imparting a good feeling to a target in which an oily component is present and forming a film having excellent washing durability, the component (B) preferably contains one or more selected from the group consisting of the components (B1) to (B6), and more preferably contains one or more selected from the group consisting of the component (B1) and the component (B2). The total content of the component (B1) and the component (B2) in the component (B) is preferably 50 mass% or more, more preferably 70 mass% or more, even more preferably 80 mass% or more, and still more preferably 90 mass% or more, and is 100 mass% or less.

More specifically, from the viewpoint of imparting a good feeling to a target in which an oily component is present and forming a film having excellent washing durability, the component (B) preferably contains one or more selected from the group consisting of polymethylsilsesquioxane, polypropylsilsesquioxane, an (acrylates/polytrimethylsiloxymethacrylate) copolymer, and an (acrylates/dimethicone) copolymer, more preferably contains one or more selected from the group consisting of polymethylsilsesquioxane, an (acrylates/polytrimethylsiloxymethacrylate) copolymer, and an (acrylates/dimethicone) copolymer, even more preferably contains one or more selected from the group consisting of polymethylsilsesquioxane and an (acrylates/polytrimethylsiloxymethacrylate) copolymer, still more preferably contains an (acrylates/polytrimethylsiloxymethacrylate) copolymer, and is still even more preferably an (acrylates/polytrimethylsiloxymethacrylate) copolymer.

<Component (C): High-Molecular-Weight Organopolysiloxane>

The composition of the present invention contains a high-molecular-weight organopolysiloxane as the component (C).

The degree of polymerization of the component (C) is preferably 650 or more, more preferably 800 or more, even more preferably 1,200 or more, still more preferably 1,400 or more, still even more preferably 1,900 or more, and yet more preferably 2,200 or more from the viewpoint of imparting a good feeling to a target in which an oily component is present and forming a film having excellent washing durability, and is preferably 20,000 or less, more preferably 10,000 or less, even more preferably 4,500 or less, still more preferably 4,000 or less, and still even more preferably 3,900 or less from the viewpoint of availability. The degree of polymerization of the component (C) is preferably 650 or more, more preferably 650 or more and 20,000 or less, even more preferably 800 or more and 10,000 or less, still more preferably 1,200 or more and 4,500 or less, still even more preferably 1,400 or more and 4,000 or less, yet more preferably 1,900 or more and 4,000 or less, and yet even more preferably 2,200 or more and 3,900 or less.

More specifically, the component (C) is preferably an organopolysiloxane represented by General Formula (1) described below.

[Chem. 7]

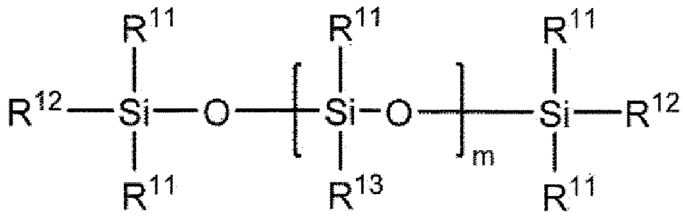

(1)

(In the formula, $R^{11}$ each independently represents a hydrocarbon group having 1 or more and 6 or less carbons, and $R^{12}$ each independently represents a hydroxy group, an alkoxy group having 1 or more and 6 or less carbons, or a hydrocarbon group having 1 or more and 6 or less carbons, $R^{13}$ represents a hydrocarbon group having 1 or more and 6 or less carbons, or a primary to tertiary amino group-containing group, m represents a degree of polymerization and is a number of 650 or more, and m pieces of $R^{13}$ may be the same or different.)

In General Formula (1), the hydrocarbon group of $R^{11}$ may be either an aliphatic group or an aromatic group, and examples thereof include an alkyl group, an alkenyl group, and a phenyl group. The alkyl group and the alkenyl group may be linear or branched.

Among the above, $R^{11}$ is preferably an alkyl group having 1 or more and 6 or less carbons or a phenyl group, more preferably an alkyl group having 1 or more and 3 or less carbons or a phenyl group, even more preferably a methyl group or a phenyl group, and still more preferably a methyl group.

In General Formula (1), $R^{12}$ each independently represents a hydroxy group, an alkoxy group having 1 or more and 6 or less carbons, or a hydrocarbon group having 1 or more and 6 or less carbons.

Examples of the alkoxy group of $R^{12}$ include a methoxy group, an ethoxy group, a propoxy group, and a butoxy group.

The hydrocarbon group of $R^{12}$ is the same as that of $R^{11}$, and is preferably an alkyl group having 1 or more and 6 or less carbons or a phenyl group, more preferably an alkyl group having 1 or more and 3 or less carbons or a phenyl group, even more preferably a methyl group or a phenyl group, and still more preferably a methyl group.

From the viewpoint of imparting a good feeling to a target in which an oily component is present and forming a film having excellent washing durability, $R^{12}$ is preferably a hydroxy group, an alkyl group having 1 or more and 6 or less carbons, or a phenyl group, more preferably a hydroxy group, an alkyl group having 1 or more and 3 or less carbons, or a phenyl group, even more preferably a hydroxy group, a methyl group, or a phenyl group, and still more preferably a methyl group.

In General Formula (1), $R^{13}$ represents a hydrocarbon group having 1 or more and 6 or less carbons or a primary to tertiary amino group-containing group.

The hydrocarbon group of $R^{13}$ is the same as that of $R^{11}$, and is preferably an alkyl group having 1 or more and 6 or less carbons or a phenyl group, more preferably an alkyl group having 1 or more and 3 or less carbons or a phenyl group, even more preferably a methyl group or a phenyl group, and still more preferably a methyl group.

The primary to tertiary amino group-containing group (hereinafter, also referred to simply as "amino group-containing group") of $R^{13}$ is preferably a group represented by $-N(R^{14})_2$, $-NR^{14}(CH_2)_pN(R^{14})_2$, or $-NR^{14}(CH_2)_pN(R^{15})CO-R^{16}$. Here, $R^{14}$ represents a hydrogen atom or a monovalent hydrocarbon group having 1 or more and 4 or less carbons, and is preferably a hydrogen atom, a methyl group, or an ethyl group. $R^{15}$ represents a monovalent hydrocarbon group having 1 or more and 4 or less carbons, and is preferably a methyl group or an ethyl group. $R^{16}$ represents a monovalent hydrocarbon group having 1 or more and 4 or less carbons. p represents a number of 2 or more and 6 or less, preferably a number of 2 or more and 4 or less.

The amino group-containing group of $R^{13}$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$.

From the viewpoint of imparting a good feeling to a target in which an oily component is present and forming a film having excellent washing durability, $R^{13}$ is preferably a hydrocarbon group having 1 or more and 6 or less carbons, more preferably an alkyl group having 1 or more and 6 or less carbons or a phenyl group, even more preferably an alkyl group having 1 or more and 3 or less carbons or a phenyl group, still more preferably a methyl group or a phenyl group, and still even more preferably a methyl group.

In General Formula (1), m represents a degree of polymerization and is a number of 650 or more. From the viewpoint of imparting a good feeling to a target in which an oily component is present and forming a film having excellent washing durability, m is preferably 800 or more, more preferably 1,200 or more, even more preferably 1,400 or more, still more preferably 1,900 or more, and still even more preferably 2,200 or more. From the viewpoint of availability, m is preferably 20,000 or less, more preferably 10,000 or less, even more preferably 4,500 or less, still more preferably 4,000 or less, and still even more preferably 3,900 or less. In General Formula (1), m is 650 or more, preferably 650 or more and

20,000 or less, more preferably 800 or more and 10,000 or less, even more preferably 1,200 or more and 4,500 or less, still more preferably 1,400 or more and 4,000 or less, still even more preferably 1,900 or more and 4,000 or less, and yet more preferably 2,200 or more and 3,900 or less.

The viscosity of the component (C) is preferably 5,000 $mm^2/s$ or more, more preferably 10,000 $mm^2/s$ or more, even more preferably 50,000 $mm^2/s$ or more, still more preferably 100,000 $mm^2/s$ or more, still even more preferably 450,000 $mm^2/s$ or more, and yet more preferably 1,000,000 $mm^2/s$ or more, from the viewpoint of imparting a good feeling to a target in which an oily component is present and forming a film having excellent washing durability. From the viewpoint of availability, the viscosity is preferably 140,000,000 $mm^2/s$ or less, more preferably 50,000,000 $mm^2/s$ or less, and even more preferably 40,000,000 $mm^2/s$ or less. The viscosity of the component (C) is preferably 5,000 $mm^2/s$ or more and 140,000,000 $mm^2/s$ or less, more preferably 10,000 $mm^2/s$ or more and 140,000,000 $mm^2/s$ or less, even more preferably 10,000 $mm^2/s$ or more and 140,000,000 $mm^2/s$ or less, still more preferably 50,000 $mm^2/s$ or more and 140,000,000 $mm^2/s$ or less, still even more preferably 100,000 $mm^2/s$ or more and 50,000,000 $mm^2/s$ or less, yet more preferably 450,000 $mm^2/s$ or more and 40,000,000 $mm^2/s$ or less, and yet even more preferably 1,000,000 $mm^2/s$ or more and 40,000,000 $mm^2/s$ or less.

The viscosity is a value measured at 25°C in accordance with JIS Z8803:2011 "Methods for viscosity measurement of liquid". For example, the viscosity can be measured with a viscometer appropriately selected from a capillary viscometer, a falling ball viscometer, a rotational viscometer, and a vibration viscometer. In a case where the viscosity exceeds the usual measurement range of the viscometer, it can be determined from a diluted solution of the component (C) by the following method.

A toluene solution of the component (C) having a concentration of 1 g/100 mL is prepared, and a specific viscosity ηsp (25°C) is determined by Mathematical Formula (1) described below. Next, an intrinsic viscosity [η] is determined by substituting ηsp into the Huggins relational expression shown in Mathematical Formula (2) described below. Further, [η] is substituted into the A. Kolorlov expression shown in Mathematical Formula (3) described below, to determine a molecular weight M. Finally, the viscosity η of the component (C) can be determined by substituting M into the A. J. Barry expression shown in Mathematical Formula (4) described below (see, for example, Silicone Oil KF-96 Performance Test Result 4.2, Shin-Etsu Chemical Co., Ltd.).

$$\eta sp = (\eta/\eta 0) - 1 \quad (1)$$

(η0: the viscosity of toluene, η: the viscosity of the solution)

$$\eta sp = [\eta] + K'[\eta]^2 \qquad (2)$$

(K': Huggins constant used is a value described in Nakamuta, J. Chem, Soc. Japan, 77588 [1956])

$$[\eta] = 0.215 \times 10^{-4} M^{0.65} \quad (3)$$

$$\log\eta = 1.00 + 0.0123 M^{0.5} \quad (4)$$

Examples of commercially available products of dimethylpolysiloxane to be used as the component (C) include KF-96H-10,000 cs (viscosity 10,000 $mm^2/s$), KF-96H-12,500 cs (viscosity 12,500 $mm^2/s$), KF-96H-30,000 cs (viscosity 30,000 $mm^2/s$), KF-96H-50,000 cs (viscosity 50,000 $mm^2/s$), KF-96H-60,000 cs (viscosity 60,000 $mm^2/s$), KF-96H-100,000 cs (viscosity 100,000 $mm^2/s$), KF-96H-300,000 cs (viscosity 300,000 $mm^2/s$), KF-96H-500,000 cs (viscosity 500,000 $mm^2/s$), KF-96H-1,000,000 cs (viscosity 1,000,000 $mm^2/s$), X-21-5686 (viscosity 3,000,000 $mm^2/s$, 30 mass% isododecane solution), and X-25-9074 (viscosity 30,000,000 $mm^2/s$, 30 mass% isododecane solution) (all available from Shin-Etsu Chemical Co., Ltd.), and TSF451-100MA (viscosity 1,000,000 $mm^2/s$), TSE200A, and Silsoft B3020 (viscosity 20,000,000 $mm^2/s$, 20 mass% isododecane solution) (all available from Momentive Performance Materials Inc.).

Examples of commercially available products of aminopropylmethylpolysiloxane to be used as the component (C) include KF-8017 (10 mass% low-viscosity dimethylpolysiloxane solution), KF-8018 (10 mass% cyclopentasiloxane solution), and KF-8020 (20 mass% low-viscosity dimethylpolysiloxane solution) (all available from Shin-Etsu Chemical Co., Ltd.).

Examples of commercially available products of dimethiconol to be used as the component (C) include X-21-5613 (20 mass% low-viscosity dimethylpolysiloxane solution), X-21-5666 (30 mass% cyclopentasiloxane solution), X-21-5847, and X-21-5849 (all available from Shin-Etsu Chemical Co., Ltd.).

The component (C) is preferably one or more selected from the group consisting of dimethylpolysiloxane,

methylphenylpolysiloxane, aminopropylmethylpolysiloxane, and dimethiconol, each of which has a degree of polymerization falling within the above range, and more preferably dimethylpolysiloxane having a degree of polymerization falling within the above range, from the viewpoint of imparting a good feeling to a target in which an oily component is present and forming a film having excellent washing durability.

<Component (D): Organopolysiloxane other than Components (A) to (C), Having Polyalkylene Oxide Moiety and Cationic Group>

The composition of the present invention contains, as the component (D), an organopolysiloxane other than the components (A) to (C), the organopolysiloxane having a polyalkylene oxide moiety and a cationic group.

Examples of the component (D) include silicones having a polyalkylene oxide moiety and a cationic group in a main chain or a side chain, such as dimethylpolysiloxane (dimethicone) and methylphenylpolysiloxane.

The cationic group contained in the component (D) refers to a cationic group or a group which can be ionized to become a cationic group. Specific examples thereof include a primary amino group, a secondary amino group, a tertiary amino group, and a quaternary ammonium group. The cationic group contained in the component (D) is preferably one or more selected from the group consisting of a primary amino group, a secondary amino group, and a tertiary amino group.

More specifically, the component (D) is more preferably one or more selected from the group consisting of a silicone (D1) having a repeating unit represented by General Formula (2) described below and a silicone (D2) having a repeating unit represented by General Formula (3) described below.

In the following description, the silicone (D1) having a repeating unit represented by General Formula (2) is also referred to as "component (D1)", and the silicone (D2) having a repeating unit represented by General Formula (3) is also referred to as "component (D2)".

(Component (D1))

The component (D1) is a silicone having a repeating unit represented by General Formula (2) described below.

[Chem. 8]

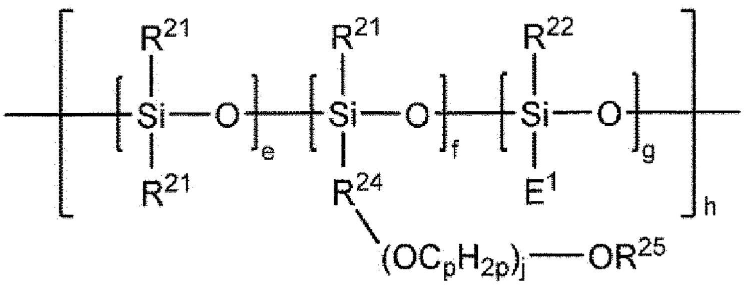

(2)

In Formula (2), $R^{21}$ represents a monovalent hydrocarbon group having 1 or more and 6 or less carbons. $R^{22}$ represents either $R^{21}$ or $E^1$. $E^1$ represents a monovalent group represented by $-R^{23}-Z^1$ (wherein $R^{23}$ represents a divalent hydrocarbon group having 1 or more and 6 or less carbons, and $Z^1$ represents a primary to tertiary amino group-containing group). $R^{24}$ represents a divalent hydrocarbon group having 1 or more and 6 or less carbons, and $R^{25}$ represents a hydrogen atom or a monovalent hydrocarbon group having 1 or more and 4 or less carbons.

e represents a number of 1 or more and 50 or less, f represents a number of 1 or more and 50 or less, g represents a number of 1 or more and 50 or less, h represents a number of 1 or more, and j represents a number of 2 or more and 100 or less. p represents a number of 2 or more and 10 or less. The bonding order of structural units in the parentheses is not limited, and the bonding form may be a block form or a random form. j pieces of $OC_pH_{2p}$ may be the same or different. Further, a plurality of $R^{21}$, $R^{22}$, $R^{24}$, $R^{25}$ and $E^1$ may be the same or different.

In General Formula (2), $R^{21}$ is a monovalent hydrocarbon group having 1 or more and 6 or less carbons, preferably an alkyl group having 1 or more and 6 or less carbons or a phenyl group, more preferably a methyl group or an ethyl group, and even more preferably a methyl group. $R^{22}$ represents either $R^{21}$ or $E^1$, and is preferably $R^{21}$.

In General Formula (2), $E^1$ represents a monovalent group represented by $-R^{23}-Z^1$ (wherein $R^{23}$ represents a divalent hydrocarbon group having 1 or more and 6 or less carbons, and $Z^1$ represents a primary to tertiary amino group-containing group). $R^{23}$ is preferably a divalent hydrocarbon group having 2 or more and 4 or less carbons, and more preferably an ethylene group, a trimethylene group, a propylene group, or a tetramethylene group.

$Z^1$ is a primary to tertiary amino group-containing group, and is preferably a group represented by $-N(R^{26})_2$, $-NR^{26}(CH_2)_qN(R^{26})_2$, or $-NR^{26}(CH_2)_qN(R^{27})CO-R^{28}$. Here, $R^{26}$ represents a hydrogen atom or a monovalent hydrocarbon

group having 1 or more and 4 or less carbons, and is preferably a hydrogen atom, a methyl group, or an ethyl group. $R^{27}$ represents a monovalent hydrocarbon group having 1 or more and 4 or less carbons, and is preferably a methyl group or an ethyl group. $R^{28}$ represents a monovalent hydrocarbon group having 1 to 4 carbons. q represents a number of 2 or more and 6 or less, and is preferably 2 or more and 4 or less.

In General Formula (2), the $E^1$ group is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In General Formula (2), $R^{24}$ represents a divalent hydrocarbon group having 1 or more and 6 or less carbons, and is preferably a divalent hydrocarbon group having 2 or more and 4 or less carbons, and more preferably an ethylene group, a trimethylene group, a propylene group, or a tetramethylene group.

$R^{25}$ represents a hydrogen atom or a monovalent hydrocarbon group having 1 or more and 4 or less carbons, and is preferably a methyl group or an ethyl group.

In General Formula (2), e represents a number of 1 or more and 50 or less, f represents a number of 1 or more and 50 or less, g represents a number of 1 or more and 50 or less, h represents a number of 1 or more, and j represents a number of 2 or more and 100 or less. p represents a number of 2 or more and 10 or less, and is preferably 2 or more and 6 or less, and more preferably 2 or more and 4 or less.

Among them, the component (D1) is more preferably represented by General Formula (2-1) described below.

[Chem. 9]

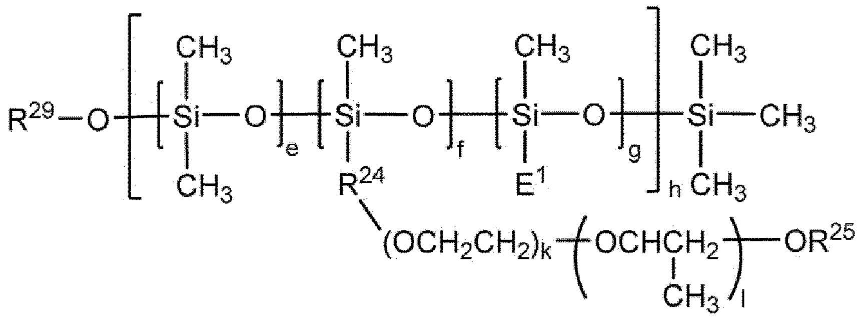

(2-1)

In Formula (2-1), $E^1$, $R^{24}$, $R^{25}$, e, f, g, and h are the same as described above. $R^{29}$ represents a monovalent hydrocarbon group having 1 or more and 4 or less carbons or a trimethylsilyl group. k represents a number of 1 or more and 50 or less, and l represents a number of 0 or more and 50 or less, and is preferably a number of 1 or more and 50 or less.

$R^{29}$ is preferably a methyl group, an ethyl group, or a trimethylsilyl group, and more preferably a trimethylsilyl group.

As the component (D1), one type may be used or two or more types may be used in combination.

A commercially available aminopolyether-modified silicone can also be used as the component (D1). Examples thereof include ABIL Soft AF100 (aminopolyether-modified silicone (methoxy PEG/PPG-7/3 aminopropyl dimethicone) represented by General Formula (2-1)) (available from Evonik Industries AG).

(Component (D2))

The component (D2) is a silicone having a repeating unit represented by General Formula (3) described below.

[Chem. 10]

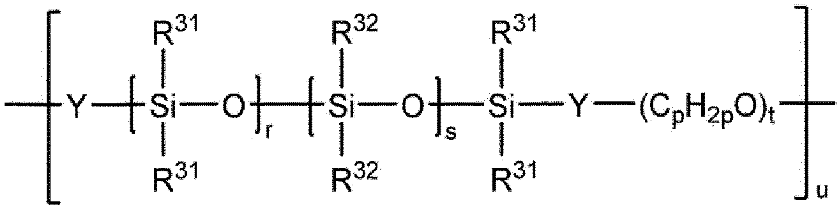

(3)

In Formula (3), $R^{31}$ represents a monovalent hydrocarbon group having 1 or more and 6 or less carbons. $R^{32}$ represents either $R^{31}$ or $E^2$. $E^2$ represents a monovalent group represented by $-R^{33}-Z^2$ (wherein $R^{33}$ represents a single bond or a divalent hydrocarbon group having 1 or more and 20 or less carbons, and $Z^2$ represents a primary to tertiary amino group-containing group). Y represents a single bond or a divalent group having 1 or more and 12 or less carbons. In a case where all $R^{32}$s are $R^{31}$, at least one Y is a divalent group containing an amino group. In a case where all Ys are a

divalent group containing no amino group, at least one $R^{32}$ is $E^2$.

r represents a number of 1 or more, s represents a number of 1 or more, t represents a number of 2 or more and 100 or less, and u represents a number of 1 or more. p is as described above and represents a number of 2 or more and 10 or less. The bonding order of structural units in the parentheses is not limited, and the bonding form may be a block form or a random form. t pieces of $C_pH_{2p}O$ may be the same or different. Further, a plurality of $R^{31}$, $R^{32}$, $E^2$, and Y may be the same or different.

In General Formula (3), $R^{31}$ represents a monovalent hydrocarbon group having 1 or more and 6 or less carbons, and is each independently preferably an alkyl group having 1 or more and 6 or less carbons or a phenyl group, more preferably a methyl group or an ethyl group, and even more preferably a methyl group.

In General Formula (3), $R^{32}$ represents either $R^{31}$ or $E^2$. $E^2$ represents a monovalent group represented by $-R^{33}-Z^2$ (wherein $R^{33}$ represents a single bond or a divalent hydrocarbon group having 1 or more and 20 or less carbons).

$R^{33}$ is preferably a divalent hydrocarbon group having 1 or more and 20 or less carbons, more preferably an alkylene group having 1 or more and 20 or less carbons, even more preferably a linear or branched alkylene group having 1 or more and 6 or less carbons, still more preferably a methylene group, an ethylene group, a trimethylene group, a propylene group, a tetramethylene group, or a hexamethylene group, and still even more preferably a trimethylene group or a propylene group.

$Z^2$ is a primary to tertiary amino group-containing group, and is preferably an amino group-containing group represented by $-N(R^{34})_2$, $-NR^{34}(CH_2)_qN(R^{34})_2$, or $-NR^{34}(CH_2)_qN(R^{35})CO-R^{36}$. Here, $R^{34}$ and $R^{35}$ each independently represent a hydrogen atom or an alkyl group having 1 or more and 3 or less carbons, and are preferably a hydrogen atom or a methyl group. $R^{36}$ represents an alkyl group having 1 or more and 3 or less carbons. q represents a number of 1 or more and 6 or less, and is preferably a number of 2 or more and 4 or less.

In General Formula (3), the $E^2$ group is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In General Formula (3), Y is a single bond or a divalent group having 1 or more and 12 or less carbons, and the divalent group having 1 or more and 12 or less carbons is preferably a divalent group represented by $-R^{37}-O-CH_2-CH(OH)-CH_2-N(R^{38})-R^{39}-O-$, which is a divalent group containing an alkylene group having 1 or more and 6 or less carbons, an alkyleneoxy group having 1 or more and 6 or less carbons, or an amino group. Here, $R^{37}$ and $R^{39}$ each independently represent an alkylene group having 1 or more and 6 or less carbons, and are preferably an alkylene group having 2 or more and 4 or less carbons, and more preferably a propylene group. $R^{38}$ represents a hydrogen atom or an alkyl group having 1 or more and 3 or less carbons.

In Y of General Formula (3), the alkylene group having 1 or more and 6 or less carbons and the alkylene group in the alkyleneoxy group having 1 or more and 6 or less carbons are preferably an ethylene group, a propylene group, a trimethylene group, an n-butylene group (tetramethylene group), or an isobutylene group, and more preferably an n-butylene group or an isobutylene group. The isobutylene group referred to herein includes $-CH(CH_3)CH_2CH_2-$, $-CH_2CH(CH_3)CH_2-$, and $-CH_2CH_2CH(CH_3)-$.

In General Formula (3), r represents a number of 1 or more, s represents a number of 1 or more, t represents a number of 2 or more and 100 or less, and u represents a number of 1 or more. r is preferably a number of 1 or more and 1000 or less, and more preferably a number of 2 or more and 200 or less. s is preferably a number of 1 or more and 100 or less, t is preferably a number of 4 or more and 80 or less, and more preferably a number of 10 or more and 50 or less, and u is preferably a number of 1 or more and 300 or less, and more preferably a number of 1 or more and 150 or less.

In General Formula (3), p represents a number of 2 or more and 10 or less, and is preferably 2 or more and 6 or less, and more preferably 2 or more and 4 or less.

The component (D2) is more preferably one or more selected from the group consisting of a silicone having a structure represented by General Formula (3-1) described below and a silicone having a structure represented by General Formula (3-2) described below.

[Chem. 11]

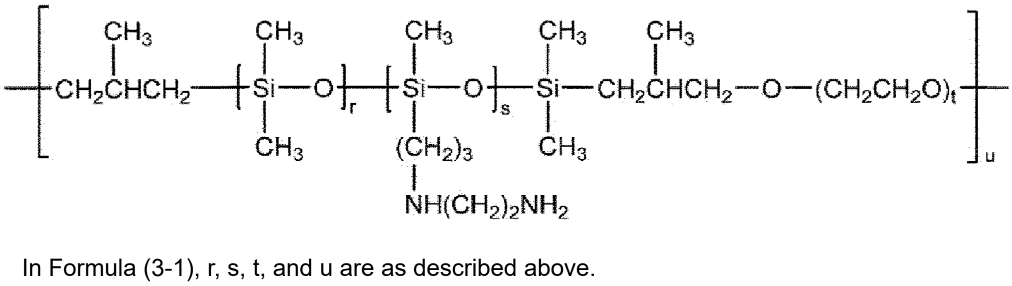

(3-1)

In Formula (3-1), r, s, t, and u are as described above.

[Chem. 12]

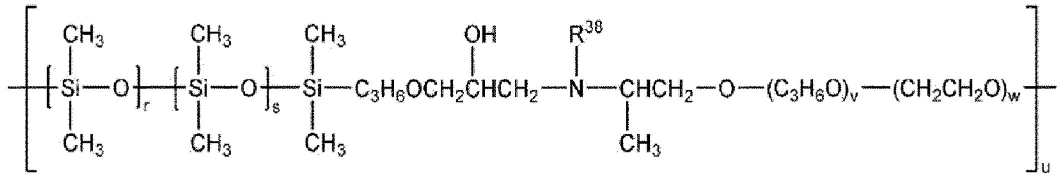

(3-2)

In Formula (3-2), $R^{38}$, r, s, and u are as described above. v represents a number of 0 or more and 50 or less, preferably 2 or more and 50 or less, and w represents a number of 2 or more and 100 or less. v + w is a number of 2 or more and 100 or less, preferably 4 or more and 80 or less, and more preferably 10 or more and 50 or less.

As the component (D2), one type may be used or two or more types may be used in combination.

A commercially available aminopolyether-modified silicone can also be used as the component (D2). Examples of the aminopolyether-modified silicone having a structure represented by General Formula (3-1) include DOWSIL SS-3588 Fluid (80 mass% ethanol solution of (bisisobutyl PEG-15/amodimethicone) copolymer), DOWSIL SILSTYLE 104 ((bisisobutyl PEG-14/amodimethicone) copolymer), and DOWSIL SILSTYLE 201 ((bisisobutyl PEG-14/amodimethicone) copolymer) (all available from Dow Toray Co., Ltd.). Examples of the aminopolyether-modified silicone having a structure represented by General Formula (3-2) include Silsoft A+ (PEG-40/PPG-8 methylaminopropyl/hydroxypropyl dimethicone copolymer) (available from Momentive Performance Materials Inc.).

Examples of the aminopolyether-modified silicone having a structure represented by General Formula (3) other than the above include DOWSIL SILSTYLE 401 ((bisisobutyl PEG/PPG-20/35/amodimethicone) copolymer) (available from Dow Toray Co., Ltd.).

As the component (D), one type or two or more types of the above-described components may be used. Among them, from the viewpoint of adsorbability onto the surface of a keratin substance, the component (D) is preferably a silicone (D2) having a repeating unit represented by General Formula (3), more preferably one or more selected from the group consisting of a silicone having a structure represented by General Formula (3-1) and a silicone having a structure represented by General Formula (3-2), and even more preferably a silicone having a structure represented by General Formula (3-1).

<Content>

The content of the component (A) in the composition is preferably 0.5 mass% or more, more preferably 1 mass% or more, even more preferably 1.5 mass% or more, still more preferably 2 mass% or more, still even more preferably 3 mass% or more, yet more preferably 5 mass% or more, yet even more preferably 7 mass% or more, and yet still even more preferably 10 mass%, from the viewpoint of improving film formability and washing durability, and is preferably 30 mass% or less, more preferably 25 mass% or less, even more preferably 20 mass% or less, and still more preferably 15 mass% or more, from the viewpoint of imparting a good feeling to a target. The content of the component (A) in the composition is preferably 0.5 mass% or more and 30 mass% or less, more preferably 1 mass% or more and 25 mass% or less, even more preferably 2 mass% or more and 20 mass% or less, still more preferably 3 mass% or more and 20 mass% or less, still even more preferably 5 mass% or more and 20 mass% or less, yet more preferably 7 mass% or more and 15 mass% or less, and yet even more preferably 10 mass% or more and 15 mass% or less.

The content of the component (B) in the composition is preferably 0.1 mass% or more, more preferably 0.5 mass% or more, even more preferably 1.0 mass% or more, even more preferably 2.0 mass% or more, still more preferably 3.0 mass% or more, still even more preferably 5.0 mass% or more, yet more preferably 7.0 mass% or more, and is preferably 30 mass% or less, more preferably 25 mass% or less, even more preferably 20 mass% or less, still more preferably 15 mass% or less, and still even more preferably 12 mass% or less, from the viewpoint of imparting a good feeling to a target in which an oily component is present and forming a film having excellent washing durability. The content of the component (B) in the composition is preferably 0.1 mass% or more and 30 mass% or less, more preferably 0.5 mass% or more and 25 mass% or less, even more preferably 1.0 mass% or more and 20 mass% or less, still more preferably 2.0 mass% or more and 15 mass% or less, still even more preferably 3.0 mass% or more and 15 mass% or less, yet more preferably 5.0 mass% or more and 15 mass% or less, and yet even more preferably 7.0 mass% or more and 12 mass% or less.

The total content of the component (A) and the component (B) in the composition is preferably 0.6 mass% or more, more preferably 1.0 mass% or more, even more preferably 2.0 mass% or more, still more preferably 3.0 mass% or more, still even more preferably 5.0 mass% or more, yet more preferably 10 mass% or more, yet even more preferably 15 mass% or more, yet still even more preferably 17 mass% or more, and yet still even more preferably 20 mass% or more, from the viewpoint of improving film formability and washing durability. The total content is preferably 60 mass% or less,

more preferably 50 mass% or less, even more preferably 40 mass% or less, still more preferably 35 mass% or less, and still even more preferably 27 mass% or less, from the viewpoint of imparting a good feeling to a target in which an oily component is present and forming a film having excellent washing durability. The total content of the component (A) and the component (B) in the composition is preferably 0.6 mass% or more and 60 mass% or less, more preferably 1.0 mass% or more and 50 mass% or less, even more preferably 2.0 mass% or more and 40 mass% or less, still more preferably 3.0 mass% or more and 40 mass% or less, still even more preferably 5.0 mass% or more and 40 mass% or less, yet more preferably 10 mass% or more and 40 mass% or less, yet even more preferably 15 mass% or more and 35 mass% or less, yet still even more preferably 17 mass% or more and 27 mass% or less, and yet still even more preferably 20 mass% or more and 27 mass% or less.

The ratio [(A)/{(A) + (B)}] of the mass content of the component (A) to the total mass content of the component (A) and the component (B) in the composition is preferably 10% or more, more preferably 20% or more, even more preferably 30% or more, still more preferably 40% or more, and still even more preferably 50% or more, from the viewpoint of improving film formability and washing durability. From the viewpoint of imparting a good feeling to a target in which an oily component is present, the ratio is preferably 95% or less, more preferably 90% or less, even more preferably 80% or less, still more preferably 70% or less, and still even more preferably 65% or less. The ratio [(A)/{(A) + (B)}] of the mass content of the component (A) to the total mass content of the component (A) and the component (B) in the composition is preferably 10% or more and 95% or less, more preferably 20% or more and 90% or less, even more preferably 30% or more and 80% or less, still more preferably 40% or more and 70% or less, and still even more preferably 50% or more and 65% or less.

The content of the component (C) in the composition is preferably 0.01 mass% or more, more preferably 0.02 mass% or more, even more preferably 0.05 mass% or more, still more preferably 0.1 mass% or more, still even more preferably 0.2 mass% or more, yet more preferably 0.5 mass% or more, and yet even more preferably 1.0 mass% or more, from the viewpoint of improving washing durability. Meanwhile, the content is preferably 10 mass% or less, more preferably 8.0 mass% or less, even more preferably 5.0 mass% or less, still more preferably 4.0 mass% or less, and still even more preferably 3.5 mass% or less, from the viewpoint of suppressing stickiness and imparting a good feeling. The content of the component (C) in the composition is preferably 0.01 mass% or more and 10 mass% or less, more preferably 0.02 mass% or more and 8.0 mass% or less, even more preferably 0.05 mass% or more and 5.0 mass% or less, still more preferably 0.1 mass% or more and 4.0 mass% or less, still even more preferably 0.2 mass% or more and 4.0 mass% or less, yet more preferably 0.5 mass% or more and 3.5 mass% or less, and yet even more preferably 1.0 mass% or more and 3.5 mass% or less.

The ratio [{(A) + (B)}/{(A) + (B) + (C)}] of the total mass content of the component (A) and the component (B) to the total mass content of the components (A) to (C) in the composition is preferably 50% or more, more preferably 60% or more, even more preferably 70% or more, still more preferably 80% or more, and still even more preferably 90% or more, and is preferably 99.5% or less, more preferably 99% or less, even more preferably 98% or less, and still more preferably 95% or less, from the viewpoint of imparting a good feeling to a target in which an oily component is present and forming a film having excellent washing durability. The ratio [{(A) + (B)}/{(A) + (B) + (C)}] of the total mass content of the component (A) and the component (B) to the total mass content of the components (A) to (C) in the composition is preferably 50% or more and 99.5% or less, more preferably 60% or more and 99% or less, even more preferably 70% or more and 98% or less, still more preferably 80% or more and 98% or less, and still even more preferably 90% or more and 95% or less.

The content of the component (D) in the composition is preferably 0.01 mass% or more, more preferably 0.02 mass% or more, even more preferably 0.05 mass% or more, still more preferably 0.1 mass% or more, and still even more preferably 0.2 mass% or more, from the viewpoint of improving washing durability. Meanwhile, the content is preferably 8.0 mass% or less, more preferably 5.0 mass% or less, even more preferably 3.0 mass% or less, and still more preferably 2.5 mass% or less, from the viewpoint of suppressing stickiness and imparting a good feeling. The content of the component (D) in the composition is preferably 0.01 mass% or more and 8.0 mass% or less, more preferably 0.02 mass% or more and 5.0 mass% or less, even more preferably 0.05 mass% or more and 3.0 mass% or less, still more preferably 0.1 mass% or more and 3.0 mass% or less, and still even more preferably 0.2 mass% or more and 2.5 mass% or less.

The total content of the components (A) to (D) in the composition is preferably 1.5 mass% or more, more preferably 3.0 mass% or more, even more preferably 5.0 mass% or more, still more preferably 10 mass% or more, still even more preferably 15 mass% or more, and yet more preferably 20 mass% or more, from the viewpoint of improving film formability and washing durability. Meanwhile, the total content is preferably 70 mass% or less, more preferably 60 mass% or less, even more preferably 50 mass% or less, still more preferably 40 mass% or less, and still even more preferably 30 mass% or less, from the viewpoint of imparting a good feeling to a target in which an oily component is present. The total content of the components (A) to (D) in the composition is preferably 1.5 mass% or more and 70 mass% or less, more preferably 3.0 mass% or more and 60 mass% or less, even more preferably 5.0 mass% or more and 50 mass% or less, still more preferably 10 mass% or more and 40 mass% or less, still even more preferably 15 mass% or more and 40 mass% or less, yet more preferably 20 mass% or more and 40 mass% or less, and yet even more preferably 20 mass% or more and 30 mass% or less.

The ratio [{(A) + (B))/{(A) + (B) + (C) + (D)}] of the total mass content of the component (A) and the component (B)

to the total mass content of the components (A) to (D) in the composition is preferably 60% or more, more preferably 70% or more, even more preferably 75% or more, still more preferably 80% or more, and still even more preferably 90% or more, from the viewpoint of imparting a good feeling to a target in which an oily component is present. Meanwhile, from the viewpoint of improving washing durability, the ratio is preferably 99% or less, more preferably 98% or less, and even more preferably 95% or less. The ratio [{(A) + (B)}/{(A) + (B) + (C) + (D)}] of the total mass content of the component (A) and the component (B) to the total mass content of the components (A) to (D) in the composition is preferably 60% or more and 99% or less, more preferably 70% or more and 98% or less, even more preferably 75% or more and 95% or less, still more preferably 80% or more and 95% or less, and still even more preferably 90% or more and 95% or less.

The ratio [(D)/{(A) + (B) + (C) + (D)}] of the mass content of the component (D) to the total mass content of the components (A) to (D) in the composition is preferably 0.1% or more, more preferably 0.2% or more, even more preferably 0.3% or more, and still more preferably 0.5% or more, from the viewpoint of improving washing durability. Meanwhile, the ratio is preferably 20% or less, more preferably 15% or less, and even more preferably 12% or less, from the viewpoint of imparting a good feeling to a target in which an oily component is present. The ratio [(D)/{(A) + (B) + (C) + (D)}] of the mass content of the component (D) to the total mass content of the components (A) to (D) in the composition is preferably 0.1% or more and 20% or less, more preferably 0.2% or more and 15% or less, even more preferably 0.3% or more and 12% or less, and still more preferably 0.5% or more and 12% or less.

<Component (E): Volatile Solvent>

The composition of the present invention preferably further contains a volatile solvent as a component (E) from the viewpoint of dispersing or dissolving the components (A) to (D). The volatile solvent defined in the present invention does not include water.

Examples of the component (E) include an alcohol-based solvent, an ether-based solvent, a ketone-based solvent, an ester-based solvent, a hydrocarbon-based solvent, and a silicone-based solvent, each of which has the volatility defined above.

Examples of the alcohol-based solvent include ethanol, 1-propanol, 2-propanol, 1-butanol, 2-butanol, and benzyl alcohol.

Examples of the ether-based solvent include diethyl ether and tetrahydrofuran, and examples of the ketone-based solvent include acetone and methyl ethyl ketone.

Examples of the ester-based solvent include methyl acetate, ethyl acetate, butyl acetate, and isobutyl acetate.

Examples of the hydrocarbon-based solvent include light liquid isoparaffin (containing isoparaffin having 8 to 16 carbons as a main component), pentane, isopentane, hexane, isohexane, heptane, isoheptane, decane, isodecane, dodecane, isododecane, tridecane, isotridecane, tetradecane, and isotetradecane.

Examples of the silicone-based solvent include dimethylpolysiloxane having a viscosity of 10 $mm^2/s$ or less at 25°C, alkyl trimethicones such as methyl trimethicone, and methylphenylpolysiloxane having a viscosity of 20 $mm^2/s$ or less at 25°C. As the component (E), one type or two or more types of these may be used.

From the viewpoint of dispersing or dissolving the components (A) to (D), the component (E) is preferably one or more selected from the group consisting of alcohol-based solvents, hydrocarbon-based solvents, and silicone-based solvents, more preferably one or more selected from the group consisting of ethanol, 1-propanol, 2-propanol, 1-butanol, 2-butanol, pentane, isopentane, hexane, isohexane, heptane, isoheptane, decane, isodecane, dodecane, isododecane, tridecane, isotridecane, tetradecane, isotetradecane, light liquid isoparaffin, dimethylpolysiloxane having a viscosity at 25°C of 10 $mm^2/s$ or less, methyl trimethicone, and methylphenylpolysiloxane having a viscosity at 25°C of 20 $mm^2/s$ or less, even more preferably one or more selected from the group consisting of ethanol, isodecane, isododecane, isotetradecane, light liquid isoparaffin, dimethylpolysiloxane having a viscosity at 25°C of 5 $mm^2/s$ or less, and methyl trimethicone, still more preferably one or more selected from the group consisting of ethanol, isodecane, isododecane, isotetradecane, and light liquid isoparaffin, and still even more preferably a solvent containing isododecane.

In a case where the composition of the present invention contains the component (E), the content of the component (E) in the composition is preferably 1 mass% or more, more preferably 5 mass% or more, even more preferably 10 mass% or more, still more preferably 20 mass% or more, still even more preferably 30 mass% or more, and yet more preferably 40 mass% or more, from the viewpoint of dispersing or dissolving the components (A) to (D), and is preferably 98 mass% or less, more preferably 95 mass% or less, even more preferably 90 mass% or less, and still more preferably 80 mass% or less, from the viewpoint of adjusting a viscosity with which the composition is easily applied to a target. The content of the component (E) in the composition is preferably 1 mass% or more and 98 mass% or less, more preferably 5 mass% or more and 95 mass% or less, even more preferably 10 mass% or more and 90 mass% or less, still more preferably 20 mass% or more and 80 mass% or less, still even more preferably 30 mass% or more and 80 mass% or less, and yet more preferably 40 mass% or more and 80 mass% or less.

<Component (F): Functional Powder>

The composition of the present invention may further contain a functional powder as a component (F) depending on the product form of the composition.

In the present invention, the functional powder refers to a powder capable of imparting various characteristics such as coloring property, concealing property, gloss, UV scattering, and feeling control. For example, in a case where the composition of the present invention is a hair dye, it is preferable to contain a pigment as the component (F) from the viewpoint of imparting a desired color tone.

The pigment to be used as the component (F) need only be a pigment that is usually used in a hair dye or the like. Examples of the pigment include white inorganic pigments such as titanium oxide, zinc oxide, cerium oxide, and barium sulfate; colored inorganic pigments such as yellow iron oxide, black iron oxide, red iron oxide, carbon black, chromium oxide, chromium hydroxide, Prussian blue, and ultramarine; bright powder such as titanium oxide-coated mica, titanium oxide-coated bismuth oxychloride, iron oxide-coated mica titanium, iron oxide mica, Prussian blue-treated mica titanium, carmine-treated mica titanium, bismuth oxychloride, and fish scale foil; organic pigments such as Red No. 201, Red No. 202, Red No. 205, Red No. 226, Red No. 228, Orange No. 203, Orange No. 204, Blue No. 404, and Yellow No. 401; lake pigments such as zirconium, barium, or aluminum lakes, such as Red No. 3, Red No. 104, Red No. 106, Orange No. 205, Yellow No. 4, Yellow No. 5, Green No. 3, and Blue No. 1; and composite pigments such as particulate titanium oxide-coated mica titanium, particulate zinc oxide-coated mica titanium, barium sulfate-coated mica titanium, titanium oxide-containing silicon dioxide, and zinc oxide-containing silicon dioxide. Among these, one type or two or more types may be used. Such a compound whose surface has been treated with any type of surface treatment agent may be used as the pigment. The surface treatment is not particularly limited, and can be performed in advance by any type of surface treatment, such as fluorine compound treatment, silicone treatment, silicone resin treatment, pendant treatment, silane coupling agent treatment, titanium coupling agent treatment, oil agent treatment, metal soap treatment, N-acylated lysine treatment, polyethylene glycol treatment, PVA treatment, polyacrylic acid treatment, hyaluronic acid treatment, alginic acid treatment, inorganic compound treatment, plasma treatment, or mechanochemical treatment.

A UV scattering agent that can be used as the component (F) is preferably one or more types of metal oxide powders selected from the group consisting of zinc oxide, titanium oxide, and cerium oxide. The average particle size of the particulate metal oxide powder is preferably from 10 to 500 nm, more preferably from 12 to 100 nm, and even more preferably from 15 to 50 nm, from the viewpoint of the UV protection effect. The average particle size is measured by a laser diffraction/scattering method.

In a case where the composition of the present invention contains the component (F), the content thereof in the composition is preferably 0.01 mass% or more, more preferably 0.1 mass% or more, even more preferably 0.5 mass% or more, and still more preferably 1.0 mass% or more, from the viewpoint of imparting desired performance, and is preferably 30 mass% or less, more preferably 20 mass% or less, even more preferably 15 mass% or less, and still more preferably 10 mass% or less, from the viewpoint of dispersibility in the composition and economic efficiency. The content of the component (F) in the composition is preferably 0.01 mass% or more and 30 mass% or less, more preferably 0.1 mass% or more and 20 mass% or less, even more preferably 0.5 mass% or more and 15 mass% or less, and still more preferably 1.0 mass% or more and 10 mass% or less.

<Water>

The content of water in the composition of the present invention is preferably less than 10 mass%, more preferably less than 8 mass%, even more preferably less than 6 mass%, still more preferably less than 5 mass%, and still even more preferably less than 3 mass%.

<Solid Oil>

**In** the composition of the present invention, the content of a solid oil is preferably small from the viewpoint of improving film formability, and from the viewpoint of imparting a good feeling to a target in which an oily component is present and forming a film having excellent washing durability. The solid oil is an oil agent which is solid at 25°C, and examples thereof include paraffin-based wax such as solid paraffin, polyolefin-based wax such as polyethylene wax, and beeswax. The content of the solid oil in the composition is preferably less than 50 mass%, more preferably less than 20 mass%, even more preferably less than 10 mass%, still more preferably less than 5 mass%, and still even more preferably less than 1 mass%.

<Volatile Cyclic Silicone>

In the composition of the present invention, the content of a volatile cyclic silicone such as decamethylcyclopentasiloxane is preferably small from the viewpoint of improving a drying rate. This is because the volatile cyclic silicone has a longer volatilization time than other volatile solvents, and the time required for drying after being applied onto a target tends to be longer. The content of the volatile cyclic silicone in the composition is preferably less than 5 mass%, more preferably less than 2 mass%, even more preferably less than 1 mass%, still more preferably less than 0.5 mass%, still even more preferably less than 0.1 mass%, and yet more preferably 0 mass%.

<Silicone-Based Surfactant>

In the composition of the present invention, the content of a silicone-based surfactant is preferably small from the viewpoint of improving film formability, and from the viewpoint of imparting a good feeling to a target in which an oily component is present and forming a film having excellent washing durability. The silicone-based surfactant means a surfactant having a silicone structure, typically a polysiloxane structure, and may have a hydrophilic group, a hydrophilic polymer chain, or the like in a side chain, a terminal, or the like. The content of the silicone-based surfactant in the composition is preferably less than 5 mass%, more preferably less than 2 mass%, even more preferably less than 1 mass%, still more preferably less than 0.5 mass%, and still even more preferably less than 0.1 mass%.

<Oil Gelling Agent>

In the composition of the present invention, the content of an oil gelling agent such as fatty acid dextrin, hydrophobic silica, or a lipophilic clay mineral is preferably small, from the viewpoint of improving film formability, and from the viewpoint of imparting a good feeling to a target in which an oily component is present and forming a film having excellent washing durability. The content of the oil gelling agent in the composition is preferably less than 5 mass%, more preferably less than 2 mass%, even more preferably less than 1 mass%, still more preferably less than 0.5 mass%, and still even more preferably less than 0.1 mass%.

<Nonvolatile Liquid Oil Agent>

In the composition of the present invention, the content of a nonvolatile liquid oil agent is preferably small from the viewpoint of improving film formability, and from the viewpoint of imparting a good feeling to a target in which an oily component is present and forming a film having excellent washing durability. The content of the nonvolatile liquid oil agent in the composition is preferably less than 50 mass%, more preferably less than 40 mass%, even more preferably less than 30 mass%, still more preferably less than 20 mass%, still even more preferably less than 10 mass%, yet more preferably less than 5 mass%, yet even more preferably less than 2 mass%, yet still more preferably less than 1 mass%, yet still even more preferably less than 0.5 mass%, and yet still even more preferably less than 0.1 mass%.

<Polyhydric Alcohol>

In the composition of the present invention, the content of a polyhydric alcohol is preferably small from the viewpoint of improving film formability, and from the viewpoint of imparting a good feeling to a target in which an oily component is present and forming a film having excellent washing durability. Examples of the polyhydric alcohol include propylene glycol and glycerin. The content of the polyhydric alcohol in the composition is preferably less than 5 mass%, more preferably less than 2 mass%, even more preferably less than 1 mass%, still more preferably less than 0.5 mass%, and still even more preferably less than 0.1 mass%.

<Additional Component>

The composition of the present invention may contain, besides the above-described components, a component that is usually used in cosmetic compositions, for example, an antioxidant, a fragrance, a pigment, a dye, an antiseptic, a thickener, a pH adjuster, a blood circulation promoter, a cooling agent, an antiperspirant, a bactericide, a skin activator, a humectant, an algefacient, or the like.

The composition of the present invention can be produced in accordance with a common method.

<Dosage Form and Like>

The dosage form of the composition of the present invention is not particularly limited, and the composition can be formulated in the form of liquid, paste, cream, gel, foam, spray, wax, or the like depending on the product form.

The composition of the present invention may be in the form of an emulsion-type composition such as an oil-in-water composition or a water-in-oil composition, or may be in the form of a non-emulsion-type composition. However, the

composition is preferably a non-emulsion-type composition from the viewpoint of imparting a good feeling to a target in which an oily component is present and forming a film having excellent washing durability.

[Cosmetic Kit]

The present invention can provide a cosmetic kit including two or more compositions, in which the following components (A) to (D) are contained in a cosmetic composition obtained by mixing the compositions:

(A) a film-forming polymer including an M unit represented by $(R^1)_3SiO_{1/2}$ (in which $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$;
(B) a film-forming polymer other than the component (A);
(C) a high-molecular-weight organopolysiloxane; and
(D) an organopolysiloxane other than the components (A) to (C), the organopolysiloxane having a polyalkylene oxide moiety and a cationic group.

The cosmetic kit includes two or more compositions, and can be used by mixing the compositions before use and applying them to the target. More specifically, the cosmetic kit can be used by mixing two or more compositions included in the cosmetic kit to prepare a cosmetic composition containing the components (A) to (D) before use and applying the cosmetic composition to a target through application or the like. The components (A) to (D) may be contained in any of the compositions constituting the cosmetic composition among the compositions included in the cosmetic kit.
The components (A) to (D) to be used in the cosmetic kit and preferred embodiments thereof are the same as those described in the cosmetic composition. In addition, each composition included in the cosmetic kit may contain an additional component exemplified in the cosmetic composition as necessary.
The cosmetic kit of the present invention may further include a composition containing none of the components (A) to (D). The cosmetic kit may further include a composition which does not constitute the cosmetic composition. For example, the cosmetic kit may be a multi-agent cosmetic kit including a composition 1, a composition 2, a composition 3, and a composition 4, in which the cosmetic composition containing the components (A) to (D) obtained by mixing the compositions 1 to 3 is a first agent, and the composition 4 is a second agent.

[Method for Treating Keratin Substance]

A method for treating a keratin substance with the composition of the present invention preferably includes a step of applying the composition to the keratin substance and then drying the composition from the viewpoint of rapidly forming a film.
From the viewpoint of uniform applicability, the composition of the present invention is preferably applied onto the surface of a keratin substance after being temporarily dissolved or dispersed before use. A thermodynamic means such as heating, a physical means such as mechanical application of shear stress, or a chemical means such as addition of a compatible solvent can be optionally utilized as a means for temporarily dissolving or dispersing the composition. From the viewpoint of convenience of a user, it is preferable to uniformly dissolve or disperse the composition by a physical means such as stirring or shaking.
From the viewpoint of maintaining various effects of the composition, the composition is preferably applied onto the keratin substance in a dry state through coating or the like, then dried, and used without being rinsed off. The drying of the keratin substance after application of the composition may be natural drying or drying using a device such as a hood, a hair dryer, or a straightening iron.
In a case of using the device, drying is preferably performed at a temperature of 40 to 220°C from the viewpoint of suppressing thermal damage to the keratin substance. Drying with a hood or a hair dryer is more preferable, and the drying temperature is preferably 40 to 110°C, and more preferably 50 to 90°C.
The drying time is not particularly limited as long as the film is substantially formed on the surface of the keratin substance, and is appropriately adjusted. For example, the drying time may be in a range of 10 seconds to 120 minutes.
In the treatment method of the present invention, the amount of the composition applied to the keratin substance is not particularly limited, but is usually in a range of 0.005 to 1 g per 1 g of the keratin substance.

[Method for Dyeing Hair]

The present invention can further provide a method for dyeing hair, the method including a step of applying the composition to hair and then drying the composition.
The composition is preferably a hair dye, and more preferably a hair dye containing a pigment.

The composition is applied to hair through coating or the like, then dried, and preferably used without being rinsed off. Drying of the hair after application of the composition may be natural drying or drying with a dryer or the like.
According to the present invention, hair can be temporarily or semipermanently dyed either in-bath or out-bath by the above-described simple operation. In particular, a good feeling can be imparted to even hair in which an oily component is present, color fading due to shampooing is reduced, and color sustainability is also good.
With regard to the above-described embodiments, the present invention discloses the following.

<1> A cosmetic composition containing components (A) to (D) described below:

(A) a film-forming polymer including an M unit represented by $(R^1)_3SiO_{1/2}$ (in which $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$;
(B) a film-forming polymer other than the component (A);
(C) a high-molecular-weight organopolysiloxane; and
(D) an organopolysiloxane other than the components (A) to (C), the organopolysiloxane having a polyalkylene oxide moiety and a cationic group.

<2> The composition according to <1>, wherein the component (A) is one or two or more selected from the group consisting of trimethylsiloxysilicate, phenylpropyldimethylsiloxysilicate, fluorine-modified alkyldimethylsiloxysilicate, and crosspolymers produced by crosslinking these siloxysilicates with dimethiconol or the like, preferably one or more selected from the group consisting of trimethylsiloxysilicate, trifluoropropyldimethyltrimethylsiloxysilicate, and (trimethylsiloxysilicate/dimethiconol) crosspolymers, more preferably one or more selected from the group consisting of trimethylsiloxysilicate and trifluoropropyldimethyltrimethylsiloxysilicate, and even more preferably trimethylsiloxysilicate.
<3> The composition according to <1> or <2>, wherein the component (B) contains a silicone-based polymer.
<4> The composition according to <3>, wherein a content of the silicone-based polymer in the component (B) is preferably 50 mass% or more, more preferably 70 mass% or more, even more preferably 80 mass% or more, and still more preferably 90 mass% or more, and is 100 mass% or less.
<5> The composition according to <3> or <4>, wherein the silicone-based polymer is one or more selected from the group consisting of components (B1) to (B6) described below:

(B1) a silicone resin including a T unit represented by $R^1SiO_{3/2}$ and being substantially free of a Q unit represented by $SiO_{4/2}$ (in which $R^1$ is as described above);
(B2) an acrylic silicone polymer;
(B3) a silicone-modified alicyclic structure-containing polymer;
(B4) silicone-modified pullulan;
(B5) polyurea/urethane silicone; and
(B6) oxazoline-modified silicone.

<6> The composition according to any one of <1> to <5>, wherein the component (B) preferably contains one or more selected from the group consisting of polymethylsilsesquioxane, polypropylsilsesquioxane, an (acrylates/polytrimethylsiloxymethacrylate) copolymer, and an (acrylates/dimethicone) copolymer, more preferably contains one or more selected from the group consisting of polymethylsilsesquioxane, an (acrylates/polytrimethylsiloxymethacrylate) copolymer, and an (acrylates/dimethicone) copolymer, even more preferably contains one or more selected from the group consisting of polymethylsilsesquioxane and an (acrylates/polytrimethylsiloxymethacrylate) copolymer, still more preferably contains an (acrylates/polytrimethylsiloxymethacrylate) copolymer, and still even more preferably is an (acrylates/polytrimethylsiloxymethacrylate) copolymer.
<7> The composition according to any one of <1> to <6>, wherein a degree of polymerization of the component (C) is preferably 650 or more, more preferably 650 or more and 20,000 or less, even more preferably 800 or more and 10,000 or less, still more preferably 1,200 or more and 4,500 or less, still even more preferably 1,400 or more and 4,000 or less, yet more preferably 1,900 or more and 4,000 or less, and yet even more preferably 2,200 or more and 3,900 or less.
<8> The composition according to any one of <1> to <7>, wherein the component (C) is an organopolysiloxane represented by General Formula (1) described below:

[Chem. 13]

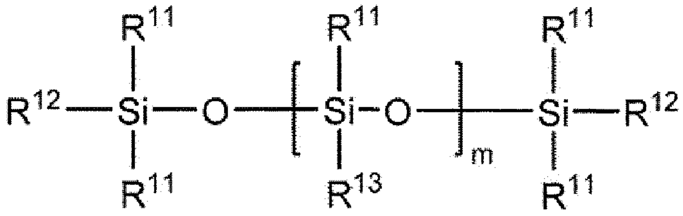

(1)

(wherein $R^{11}$ each independently represents a hydrocarbon group having 1 or more and 6 or less carbons, and $R^{12}$ each independently represents a hydroxy group, an alkoxy group having 1 or more and 6 or less carbons, or a hydrocarbon group having 1 or more and 6 or less carbons, $R^{13}$ represents a hydrocarbon group having 1 or more and 6 or less carbons, or a primary to tertiary amino group-containing group, m represents a degree of polymerization and is a number of 650 or more, and m pieces of $R^{13}$ may be the same or different).

<9> The composition according to <8>, wherein m in General Formula (1) is preferably 650 or more and 20,000 or less, more preferably 800 or more and 10,000 or less, even more preferably 1,200 or more and 4,500 or less, still more preferably 1,400 or more and 4,000 or less, still even more preferably 1,900 or more and 4,000 or less, and yet more preferably 2,200 or more and 3,900 or less.

<10> The composition according to any one of <1> to <9>, wherein a viscosity of the component (C) measured at 25°C is preferably 5,000 $mm^2/s$ or more and 140,000,000 $mm^2/s$ or less, more preferably 10,000 $mm^2/s$ or more and 140,000,000 $mm^2/s$ or less, even more preferably 10,000 $mm^2/s$ or more and 140,000,000 $mm^2/s$ or less, still more preferably 50,000 $mm^2/s$ or more and 140,000,000 $mm^2/s$ or less, still even more preferably 100,000 $mm^2/s$ or more and 50,000,000 $mm^2/s$ or less, yet even more preferably 450,000 $mm^2/s$ or more and 40,000,000 $mm^2/s$ or less, and yet still even more preferably 1,000,000 $mm^2/s$ or more and 40,000,000 $mm^2/s$ or less.

<11> The composition according to any one of <1> to <10>, wherein the component (C) is one or more selected from the group consisting of dimethylpolysiloxane, methylphenylpolysiloxane, aminopropylmethylpolysiloxane, and dimethiconol, and more preferably dimethylpolysiloxane.

<12> The composition according to any one of <1> to <11>, wherein the component (D) is one or more selected from the group consisting of a silicone (D1) having a repeating unit represented by General Formula (2) described below and a silicone (D2) having a repeating unit represented by General Formula (3) described below:

[Chem. 14]

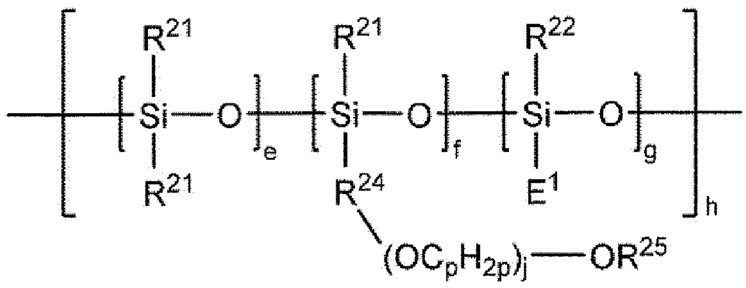

(2)

in Formula (2), $R^{21}$ represents a monovalent hydrocarbon group having 1 or more and 6 or less carbons, $R^{22}$ represents either $R^{21}$ or $E^1$, $E^1$ represents a monovalent group represented by $-R^{23}-Z^1$ (wherein $R^{23}$ represents a divalent hydrocarbon group having 1 or more and 6 or less carbons, and $Z^1$ represents a primary to tertiary amino group-containing group), $R^{24}$ represents a divalent hydrocarbon group having 1 or more and 6 or less carbons, and $R^{25}$ represents a hydrogen atom or a monovalent hydrocarbon group having 1 or more and 4 or less carbons,
e represents a number of 1 or more and 50 or less, f represents a number of 1 or more and 50 or less, g represents a number of 1 or more and 50 or less, h represents a number of 1 or more, and j represents a number of 2 or more and 100 or less, p represents a number of 2 or more and 10 or less, the bonding order of structural units in the parentheses is not limited, and the bonding form may be a block form or a random form, j pieces of $OC_pH_{2p}$ may be the same or different, and a plurality of $R^{21}$, $R^{22}$, $R^{24}$, $R^{25}$ and $E^1$ may be the same or different, and

[Chem. 15]

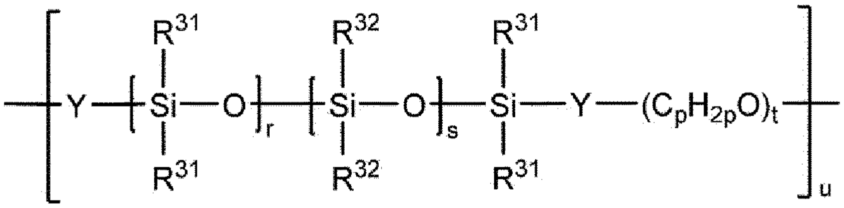

(3)

in Formula (3), $R^{31}$ represents a monovalent hydrocarbon group having 1 or more and 6 or less carbons, $R^{32}$ represents either $R^{31}$ or $E^2$, $E^2$ represents a monovalent group represented by $-R^{33}-Z^2$ (wherein $R^{33}$ represents a single bond or a divalent hydrocarbon group having 1 or more and 20 or less carbons, and $Z^2$ represents a primary to tertiary amino group-containing group), Y represents a single bond or a divalent group having 1 or more and 12 or less carbons, in a case where all $R^{32}$s are $R^{31}$, at least one Y is a divalent group containing an amino group, in a case where all Ys are a divalent group containing no amino group, at least one $R^{32}$ is $E^2$,
r represents a number of 1 or more, s represents a number of 1 or more, t represents a number of 2 or more and 100 or less, and u represents a number of 1 or more, p is as defined above and represents a number of 2 or more and 10 or less, the bonding order of structural units in the parentheses is not limited, and the bonding form may be a block form or a random form, t pieces of $C_pH_{2p}O$ may be the same or different, and a plurality of $R^{31}$, $R^{32}$, $E^2$ and Y may be the same or different.
<13> The composition according to <12>, wherein the component (D1) is represented by General Formula (2-1) described below:

[Chem. 16]

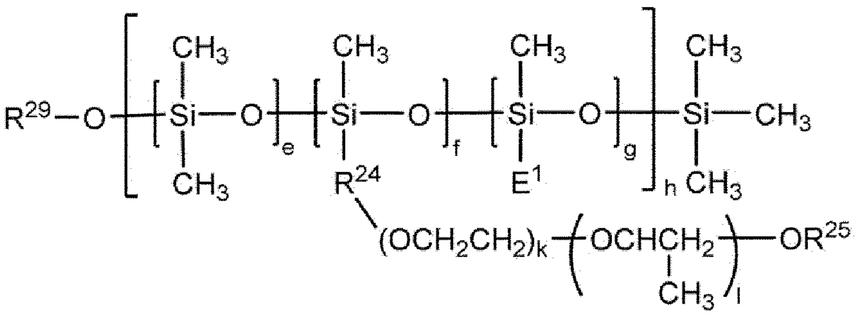

(2-1)

in Formula (2-1), $E^1$, $R^{24}$, $R^{25}$, e, f, g, and h are the same as defined above, $R^{29}$ represents a monovalent hydrocarbon group having 1 or more and 4 or less carbons or a trimethylsilyl group, and k represents a number of 1 or more and 50 or less, and l represents a number of 0 or more and 50 or less, and is preferably a number of 1 or more and 50 or less.
<14> The composition according to <12> or <13>, wherein the component (D2) is one or more selected from the group consisting of a silicone having a structure represented by General Formula (3-1) described below and a silicone having a structure represented by General Formula (3-2) described below:

[Chem. 17]

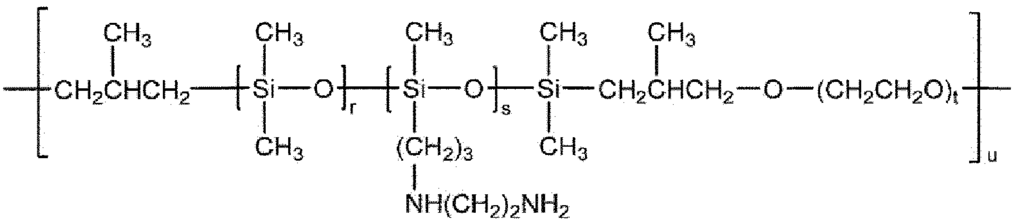

(3-1)

in Formula (3-1), r, s, t, and u are the same as described above,

[Chem. 18]

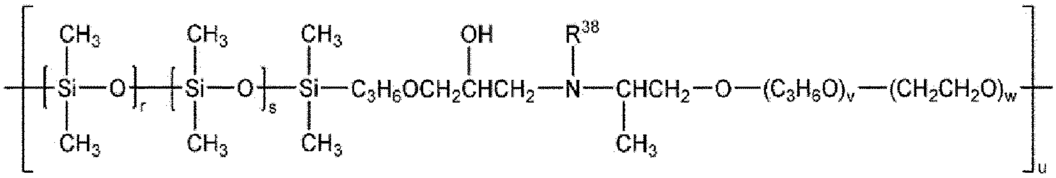

(3-2)

in Formula (3-2), $R^{38}$ represents a hydrogen atom or an alkyl group having 1 or more and 3 or less carbons, r, s and u are the same as described above, v represents a number of 0 or more and 50 or less, preferably 2 or more and 50 or less, and w represents a number of 2 or more and 100 or less, and v + w is a number of 2 or more and 100 or less, preferably 4 or more and 80 or less, and more preferably 10 or more and 50 or less.

<15> The composition according to any one of <12> to <14>, wherein the component (D) is preferably a silicone (D2) having a repeating unit represented by General Formula (3), more preferably one or more selected from the group consisting of a silicone having a structure represented by General Formula (3-1) and a silicone having a structure represented by General Formula (3-2), and even more preferably a silicone having a structure represented by General Formula (3-1).

<16> The composition according to any one of <1> to <15>, wherein a content of the component (A) in the composition is preferably 0.5 mass% or more and 30 mass% or less, more preferably 1 mass% or more and 25 mass% or less, even more preferably 2 mass% or more and 20 mass% or less, still more preferably 3 mass% or more and 20 mass% or less, still even more preferably 5 mass% or more and 20 mass% or less, yet more preferably 7 mass% or more and 15 mass% or less, and yet even more preferably 10 mass% or more and 15 mass% or less.

<17> The composition according to any one of <1> to <16>, wherein a content of the component (B) in the composition is preferably 0.1 mass% or more and 30 mass% or less, more preferably 0.5 mass% or more and 25 mass% or less, even more preferably 1.0 mass% or more and 20 mass% or less, still more preferably 2.0 mass% or more and 15 mass% or less, still even more preferably 3.0 mass% or more and 15 mass% or less, yet more preferably 5.0 mass% or more and 15 mass% or less, and yet even more preferably 7.0 mass% or more and 12 mass% or less.

<18> The composition according to any one of <1> to <17>, wherein a total content of the composition (A) and the component (B) in the composition is preferably 0.6 mass% or more and 60 mass% or less, more preferably 1.0 mass% or more and 50 mass% or less, even more preferably 2.0 mass% or more and 40 mass% or less, still more preferably 3.0 mass% or more and 40 mass% or less, still even more preferably 5.0 mass% or more and 40 mass% or less, yet more preferably 10 mass% or more and 40 mass% or less, yet even more preferably 15 mass% or more and 35 mass% or less, yet still more preferably 17 mass% or more and 27 mass% or less, and yet still even more preferably 20 mass% or more and 27 mass% or less.

<19> The composition according to any one of <1> to <18>, wherein a ratio [(A)/{(A) + (B)}] of a mass content of the component (A) to a total mass content of the component (A) and the component (B) in the composition is preferably 10% or more and 95% or less, more preferably 20% or more and 90% or less, even more preferably 30% or more and 80% or less, still more preferably 40% or more and 70% or less, and still even more preferably 50% or more and 65% or less.

<20> The composition according to any one of <1> to <19>, wherein a content of the component (C) in the composition is preferably 0.01 mass% or more and 10 mass% or less, more preferably 0.02 mass% or more and 8.0 mass% or less, even more preferably 0.05 mass% or more and 5.0 mass% or less, still more preferably 0.1 mass% or more and 4.0 mass% or less, still even more preferably 0.2 mass% or more and 4.0 mass% or less, yet more preferably 0.5 mass% or more and 3.5 mass% or less, and yet even more preferably 1.0 mass% or more and 3.5 mass% or less.

<21> The composition according to any one of <1> to <20>, wherein a ratio [{(A) + (B)}/ {(A) + (B) + (C)}] of the total mass content of the component (A) and the component (B) to a total mass content of the components (A) to (C) in the composition is preferably 50% or more and 99.5% or less, more preferably 60% or more and 99% or less, even more preferably 70% or more and 98% or less, still more preferably 80% or more and 98% or less, and still even more preferably 90% or more and 95% or less.

<22> The composition according to any one of <1> to <21>, wherein a content of the component (D) in the composition is preferably 0.01 mass% or more and 8.0 mass% or less, more preferably 0.02 mass% or more and 5.0 mass% or less, even more preferably 0.05 mass% or more and 3.0 mass% or less, still more preferably 0.1 mass% or more and 3.0 mass% or less, and still even more preferably 0.2 mass% or more and 2.5 mass% or less.

<23> The composition according to any one of <1> to <22>, wherein a total content of the components (A) to (D) in the composition is preferably 1.5 mass% or more and 70 mass% or less, more preferably 3.0 mass% or more and 60 mass% or less, even more preferably 5.0 mass% or more and 50 mass% or less, still more preferably 10 mass% or more and 40 mass% or less, still even more preferably 15 mass% or more and 40 mass% or less, yet more preferably

20 mass% or more and 40 mass% or less, and yet even more preferably 20 mass% or more and 30 mass% or less.

<24> The composition according to any one of <1> to <23>, wherein a ratio [{(A) + (B)}/{(A) + (B) + (C) + (D)}] of the total mass content of the component (A) and the component (B) to a total mass content of the components (A) to (D) in the composition is preferably 60% or more and 99% or less, more preferably 70% or more and 98% or less, even more preferably 75% or more and 95% or less, still more preferably 80% or more and 95% or less, and still even more preferably 90% or more and 95% or less.

<25> The composition according to any one of <1> to <24>, wherein a ratio [(D)/{(A) + (B) + (C) + (D)}] of a mass content of the component (D) to the total mass content of the components (A) to (D) in the composition is preferably 0.1% or more and 20% or less, more preferably 0.2% or more and 15% or less, even more preferably 0.3% or more and 12% or less, and still more preferably 0.5% or more and 12% or less.

<26> The composition according to any one of <1> to <25>, wherein the composition further contains a volatile solvent as a component (E).

<27> The composition according to <26>, wherein the component (E) is preferably one or more selected from the group consisting of an alcohol-based solvent, a hydrocarbon-based solvent, and a silicone-based solvent, more preferably one or more selected from the group consisting of ethanol, 1-propanol, 2-propanol, 1-butanol, 2-butanol, pentane, isopentane, hexane, isohexane, heptane, isoheptane, decane, isodecane, dodecane, isododecane, tridecane, isotridecane, tetradecane, isotetradecane, light liquid isoparaffin, dimethylpolysiloxane having a viscosity of 10 $mm^2/s$ or less at 25°C, methyltrimethicone, and methylphenylpolysiloxane having a viscosity of 20 $mm^2/s$ or less at 25°C, even more preferably one or more selected from the group consisting of ethanol, isodecane, isododecane, isotetradecane, light liquid isoparaffin, dimethylpolysiloxane having a viscosity of 5 $mm^2/s$ or less at 25°C, and methyltrimethicone, still more preferably one or more selected from the group consisting of ethanol, isodecane, isododecane, isotetradecane, and light liquid isoparaffin, and is still even more preferably a solvent containing isododecane.

<28> The composition according to <26> or <27>, wherein a content of the component (E) in the composition is preferably 1 mass% or more and 98 mass% or less, more preferably 5 mass% or more and 95 mass% or less, even more preferably 10 mass% or more and 90 mass% or less, still more preferably 20 mass% or more and 80 mass% or less, still even more preferably 30 mass% or more and 80 mass% or less, and yet more preferably 40 mass% or more and 80 mass% or less.

<29> The composition according to any one of <1> to <28>, wherein the composition further contains a functional powder as a component (F).

<30> The composition according to <29>, wherein a content of the component (F) in the composition is preferably 0.01 mass% or more and 30 mass% or less, more preferably 0.1 mass% or more and 20 mass% or less, even more preferably 0.5 mass% or more and 15 mass% or less, and still more preferably 1.0 mass% or more and 10 mass% or less.

<31> The composition according to any one of <1> to <30>, wherein the content of the component (A) in the composition is 5 mass% or more and 20 mass% or less.

<32> The composition according to any one of <1> to <31>, wherein the content of the component (B) in the composition is 5.0 mass% or more and 15 mass% or less.

<33> The composition according to any one of <1> to <32>, wherein a ratio [(A)/{(A) + (B)}] of the mass content of the component (A) to the total mass content of the component (A) and the component (B) in the composition is 30% or more and 80% or less.

<34> The composition according to any one of <1> to <33>, wherein the composition is preferably a cosmetic composition for a keratin substance, more preferably one or more selected from the group consisting of a skin cosmetic composition and a hair cosmetic composition, and even more preferably a hair cosmetic composition.

<35> The composition according to <34>, wherein the hair cosmetic composition is preferably a conditioner, a treatment agent, a styling agent, a hair dye, or a hair growth tonic, and more preferably a hair dye.

<36> The composition according to <34> or <35>, wherein the composition is used without being rinsed off after being applied to a keratin substance.

<37> A method for treating a keratin substance, the method including applying the composition described in any one of <1> to <36> to a keratin substance, and then drying the composition.

<38> A method for dyeing hair, the method including applying the composition described in any one of <1> to <36> to hair, and then drying the composition.

<39> A cosmetic kit including two or more compositions, wherein a cosmetic composition produced by mixing the compositions contains components (A) to (D) described below:

(A) a film-forming polymer including an M unit represented by $(R^1)_3SiO_{1/2}$ (in which $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$;

(B) a film-forming polymer other than the component (A);
(C) a high-molecular-weight organopolysiloxane; and
(D) an organopolysiloxane other than the components (A) to (C), the organopolysiloxane having a polyalkylene oxide moiety and a cationic group.

## Examples

Hereinafter, the present invention will be described with reference to Examples, but the present invention is not limited to the scope of the Examples. In the Examples, various types of measurement and evaluation were performed by methods described below.

<Preparation of Hair Bundle for Evaluation>

A human white hair (100%) bundle (available from Beaulax Co., Ltd., length: 10 cm, mass: 1 g) was washed with a plain shampoo having a composition described below, then rinsed off with warm water at 40°C, and sufficiently dried. 5 μL of model sebum (oleic acid ("LUNAC O-V" available from Kao Corporation)/squalane ("NIKKOL Squalane" available from Nippon Surfactant Industry Co., Ltd.)/olive oil ("Cropure OL" available from Croda Japan KK) = 2/2/1 (mass proportions)) was uniformly applied onto the hair bundle with a finger to prepare a hair bundle for evaluation.

(Composition of plain shampoo)

| Components | (mass%) |
|---|---|
| Sodium polyoxyethylene (2) lauryl ether sulfate (*1) | 15.5 |
| Lauric acid diethanolamide (*2) | 1.5 |
| Edetic acid tetrasodium salt | 0.3 |
| Sodium benzoate | 1.43 |
| Purified water | balance |
| Total | 100.0 |

*1: 57.4 mass% as EMAL 227 (available from Kao Corporation, active ingredient: 27 mass%)
*2: AMINON L-02 (available from Kao Corporation)

<Feeling in Presence of Sebum/in Dry State>

0.2 g of the hair cosmetic composition of each Example was applied to the hair bundle for evaluation, and then hot air was applied for 2 minutes from a position 18 cm away from the hair bundle using a dryer ("P2-D250" available from Hitachi, Ltd., setting: HIGH) to dry the hair bundle for hair treatment. The feeling (no stickiness, no stiffness, and good combability) of the treated hair bundle was sensorily evaluated by expert panelists in accordance with the following criteria, and the total score of N = 3 was calculated.

(No stickiness)

5: No stickiness is felt
4: Almost no stickiness is felt
3: Slight stickiness is felt
2: Stickiness is somewhat felt
1: Stickiness is felt

In a case where the total score of N = 3 is 6 or less, stickiness is felt and the feeling is not good. In a case where the total score is 7 to 9, slight stickiness is felt, but the feeling is somewhat good. When the total score is 10 or more, the feeling is good, and when the total score is 12 or more, the feeling is better.

(No stiffness)

5: No stiffness is felt
4: Almost no stiffness is felt
3: Slight stiffness is felt
2: Stiffness is somewhat felt
1: Stiffness is felt

In a case where the total score of N = 3 is 6 or less, stiffness is felt and the feeling is not good. In a case where the total score is 7 to 9, slight stiffness is felt, but the feeling is somewhat good. When the total score is 10 or more, the feeling is good, and when the total score is 12 or more, the feeling is better.

(Good combability)

5: Combability is felt well
4: Combability is felt
3: Combability is somewhat felt
2: Combability is rarely felt
1: No combability is felt

In a case where the total score of N = 3 is 6 or less, combing is difficult, and combability is not good. In a case where the total score is 7 to 9, combing is somewhat easy, and combability is somewhat good. When the total score is 10 or more, combability is good, and when the total score is 12 or more, combability is better.

<Shampoo Resistance (Resistance to Color Loss by Shampooing)>

0.2 g of the hair cosmetic composition of each Example was applied onto the hair bundle for evaluation, and then hot air was applied for 2 minutes from a position 18 cm away from the hair bundle using a dryer ("P2-D250" available from Hitachi, Ltd., setting: HIGH) to dry the hair bundle for hair treatment.

The treated hair bundle was subjected to color measurement in the CIE color system (L*, a*, b*) using a color difference meter ("CR-400" available from Konica Minolta, Inc.), and then a step of washing the hair bundle with warm water at 40°C using a plain shampoo having the composition described above, and drying the hair bundle was performed five times. The color of the hair bundle shampooed five times and dried was measured with the color difference meter in the same manner, and a difference ($\Delta E_2^* - \Delta E_1^*$) between $\Delta E_2^*$ after shampooing five times and $\Delta E_1^*$ after the treatment and before shampooing, which are represented by the following equations, was calculated. L*, a*, and b* were measured at six different points on the hair bundle (two points at the center of each of the regions obtained by dividing the hair bundle into three equal parts in the longitudinal direction), and the average value was calculated.

[Math. 1]

$$\Delta E_1^* = \sqrt{(L_1^* - L_0^*)^2 + (a_1^* - a_0^*)^2 + (b_1^* - b_0^*)^2}$$

[Math. 2]

$$\Delta E_2^* = \sqrt{(L_2^* - L_0^*)^2 + (a_2^* - a_0^*)^2 + (b_2^* - b_0^*)^2}$$

$L_0^*$, $a_0^*$, $b_0^*$: value of hair bundle measured before treatment
$L_1^*$, $a_1^*$, $b_1^*$: value of hair bundle measured after treatment and before shampooing
$L_2^*$, $a_2^*$, $b_2^*$: value of hair bundle measured after shampooing five times

When ($\Delta E_2^* - \Delta E_1^*$) < 15 is satisfied, it can be determined that the hair bundle after the treatment has good shampoo resistance and excellent color sustainability. It is better when ($\Delta E_2^* - \Delta E_1^*$) < 10 is satisfied, it is even better when ($\Delta E_2^* - \Delta E_1^*$) < 5 is satisfied, and it is much better when ($\Delta E_2^* - \Delta E_1^*$) < 2 is satisfied.

Examples 1 to 25 and Comparative Examples 1 to 4 (Preparation and Evaluation of Hair Cosmetic Composition)

Components shown in Tables 1 to 3 were blended at the blending proportions shown in each table and mixed until homogeneity was achieved, to prepare a hair cosmetic composition. The prepared hair cosmetic composition was used to perform various types of evaluation by the above-described methods. The results are shown in Tables 1 to 3.

Each of the blending amounts (mass%) shown in the tables is the amount of an active ingredient.

[Table 1]

Table 1

| (mass%) | | | Example 1 | Comparative Example 1 | Comparative Example 2 | Comparative Example 3 | Comparative Example 4 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|---|---|---|---|
| (A) | Trimethylsiloxysilicate | X-21-55-95 *1 | 11.6 | 6 | 6.6 | 7.5 | 10 | 4.3 | 17 | 11.6 | 11.6 | 11.6 | 11.6 | 8.7 | 9.9 | 12.1 | 4.54 | 6 | 18.15 |
| (B) | (Acrylates/dimethicone) copolymer | KP-550 *2 | 0 | 0 | 0 | 0 | 15 | 0 | 0 | 0 | 0 | 0 | 0 | 0 | 0 | 0 | 0 | 0 | 0 |
| | (Acrylates/polytrimethylsil oxymethacrylate) copolymer | F-A-4004I-D *3 | 9.7 | 0 | 0 | 0 | 0 | 17 | 4.3 | 9.7 | 9.7 | 9.7 | 9.7 | 7.2 | 8.2 | 10.1 | 3.8 | 5.1 | 15.2 |
| (C) | High-molecular-weight dimethylpolysiloxane Viscosity 20,000,000 CS | Silsoft B3020 *4 | 1.35 | 3 | 5.4 | 0 | 0 | 1.35 | 1.35 | 1.35 | 1.35 | 1.35 | 1.35 | 6.75 | 4.55 | 0.45 | 0.53 | 0.7 | 2.1 |
| (D) | PEG/amodimethicone copolymer | SS-3588 *5 | 0.35 | 3 | 0 | 1.5 | 0 | 0.35 | 0.35 | 0.05 | 0.2 | 0.8 | 2 | 0.35 | 0.35 | 0.35 | 0.13 | 0.2 | 0.55 |
| (E) | Isododecane | Marukasol R *6 | Balance | Balance | Balance | Balance | Balance | Balance | Balance | Balance | Balance | Balance | Balance | Balance | Balance | Balance | Balance | Balance | Balance |
| (F) | Red 202 | | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| Total | | | 100 | 100 | 100 | 100 | 100 | 100 | 100 | 100 | 100 | 100 | 100 | 100 | 100 | 100 | 100 | 100 | 100 |
| (A) + (B) (mass%) | | | 21.30 | 6.00 | 6.60 | 7.50 | 25.00 | 21.30 | 21.30 | 21.30 | 21.30 | 21.30 | 21.30 | 15.90 | 18.10 | 22.20 | 8.34 | 11.10 | 33.35 |
| (A) + (B) + (C) (mass%) | | | 22.65 | 9.00 | 12.00 | 7.50 | 25.00 | 22.65 | 22.65 | 22.65 | 22.65 | 22.65 | 22.65 | 22.65 | 22.65 | 22.65 | 8.87 | 11.80 | 35.45 |
| (A) + (B) + (C) + (D) (mass%) | | | 23.00 | 12.00 | 12.00 | 9.00 | 25.00 | 23.00 | 23.00 | 22.70 | 22.85 | 23.45 | 24.65 | 23.00 | 23.00 | 23.00 | 9.00 | 12.00 | 36.00 |
| (A)/{(A)+(B)} (%) | | | 54.5 | 100.0 | 100.0 | 100.0 | 40.0 | 20.2 | 79.8 | 54.5 | 54.5 | 54.5 | 54.5 | 54.7 | 54.7 | 54.5 | 54.4 | 54.1 | 54.4 |
| {(A)+(B)}/{(A)+(B)+(C)} (%) | | | 94.0 | 66.7 | 55.0 | 100.0 | 100.0 | 94.0 | 94.0 | 94.0 | 94.0 | 94.0 | 94.0 | 70.2 | 79.9 | 98.0 | 94.0 | 94.1 | 94.1 |
| {(A)+(B)}/{(A)+(B)+(C)+(D)} (%) | | | 92.6 | 50.0 | 55.0 | 83.3 | 100.0 | 92.6 | 92.6 | 93.8 | 93.2 | 90.8 | 86.4 | 69.1 | 78.7 | 96.5 | 92.7 | 92.5 | 92.6 |
| {(D)}/{(A)+(B)+(C)+(D)} (%) | | | 1.5 | 25.0 | 0.0 | 16.7 | 0.0 | 1.5 | 1.5 | 0.22 | 0.9 | 3.4 | 8.1 | 1.5 | 1.5 | 1.5 | 1.4 | 1.7 | 1.5 |

(continued)

| (mass%) | | | Example 1 | Comparative Example 1 | Comparative Example 2 | Comparative Example 3 | Comparative Example 4 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|---|---|---|---|
| Evaluation results | Feeling in presence of sebum/in dry state | No stickiness | 14 | 3 | 4 | 3 | 4 | 15 | 13 | 15 | 14 | 13 | 13 | 7 | 9 | 15 | 15 | 12 | 15 |
| | | No stiffness | 13 | 9 | 13 | 6 | 4 | 14 | 15 | 14 | 14 | 15 | 12 | 15 | 15 | 11 | 15 | 15 | 11 |
| | | Good combability | 14 | 6 | 6 | 3 | 3 | 15 | 13 | 15 | 15 | 12 | 12 | 11 | 12 | 12 | 11 | 12 | 13 |
| | | Total score | 41 | 18 | 23 | 12 | 11 | 44 | 41 | 44 | 43 | 40 | 37 | 33 | 36 | 38 | 41 | 39 | 39 |
| | Shampoo resistance | $(\Delta E_2^* - \Delta E_1^*)$ | 0.6 | - | - | - | - | 2.9 | 2.1 | 2.3 | 0.9 | 1.7 | 0.4 | -0.9 | 0.4 | 1.6 | 12.4 | 8.5 | 1.4 |

[Table 2]

Table 2

| (mass%) | | | Example 1 | Example 14 | Example 15 | Example 16 | Example 17 | Example 18 | Example 19 | Example 20 | Example 21 | Example 22 | Example 23 | Example 24 |
|---|---|---|---|---|---|---|---|---|---|---|---|---|---|---|
| (A) | Trimethylsiloxysilicate | X-21-5595 *1 | 11.6 | | 4.3 | 11.6 | 17 | 11.6 | 11.6 | 11.6 | 11.6 | 11.6 | 11.6 | 11.6 |
| | (Trimethylsiloxysilicate/dimethiconol) crosspolymer | FC-5002 *7 | | 11.6 | | | | | | | | | | |
| (B) | (Acrylates/dimethicone) copolymer | KP-550 *2 | | | 17 | 9.7 | 4.3 | | | | | | | |
| | (Acrylates/polytrimethylsiloxymethacrylate) copolymer | FA-4004ID *3 | 9.7 | 9.7 | | | | | | 9.7 | 9.7 | 9.7 | 9.7 | 9.7 |
| | Polymethylsilsesquioxane | Silform Flexible Resin *8 | | | | | | 9.7 | | | | | | |
| | (Acrylates/polytrimethylsiloxymethacrylate) copolymer | FA-4002ID *9 | | | | | | | 9.7 | | | | | |
| (C) | High-molecular-weight dimethylpolysiloxane Viscosity 100,000 CS | KF-96-H-100,000 CS *10 | | | | | | | | 1.35 | | | | |
| | High-molecular-weight dimethylpolysiloxane Viscosity 1,000,000 CS | KF-96-H-1,000,000 CS *11 | | | | | | | | | 1.35 | | | |
| | High-molecular-weight dimethylpolysiloxane Viscosity 20,000,000 CS | Silsoft B3020 *4 | 1.35 | 1.35 | 1.35 | 1.35 | 1.35 | 1.35 | 1.35 | | | | | |
| | High-molecular-weight dimethylpolysiloxane Viscosity 30,000,000 CS | (X-25-9074) *12 | | | | | | | | | | 1.35 | | |
| | Highly polymerized dimethiconol | X-21-5666 *13 | | | | | | | | | | | 1.35 | |
| | Highly polymerized amino oil | KF-8018 *14 | | | | | | | | | | | | 1.35 |
| (D) | PEG/amodimethicone copolymer | SS-3588 *5 | 0.35 | 0.35 | 0.35 | 0.35 | 0.35 | 0.35 | 0.35 | 0.35 | 0.35 | 0.35 | 0.35 | 0.35 |
| (E) | Isododecane | Marukasol R *6 | Balance | Balance | Balance | Balance | Balance | Balance | Balance | Balance | Balance | Balance | Balance | Balance |
| (F) | Red 202 | | 1.5 | 1.5 | 1.5 | 1.5 | 1.5 | 1.5 | 1.5 | 1.5 | 1.5 | 1.5 | 1.5 | 1.5 |

(continued)

| (mass%) | | | Example 1 | Example 14 | Example 15 | Example 16 | Example 17 | Example 18 | Example 19 | Example 20 | Example 21 | Example 22 | Example 23 | Example 24 |
|---|---|---|---|---|---|---|---|---|---|---|---|---|---|---|
| Total | | | 100 | 100 | 100 | 100 | 100 | 100 | 100 | 100 | 100 | 100 | 100 | 100 |
| (A) + (B) (mass%) | | | 21.30 | 21.30 | 21.30 | 21.30 | 21.30 | 21.30 | 21.30 | 21.30 | 21.30 | 21.30 | 21.30 | 21.30 |
| (A) + (B) + (C) (mass%) | | | 22.65 | 22.65 | 22.65 | 22.65 | 22.65 | 22.65 | 22.65 | 22.65 | 22.65 | 22.65 | 22.65 | 22.65 |
| (A) + (B) + (C) + (D) (mass%) | | | 23.00 | 23.00 | 23.00 | 23.00 | 23.00 | 23.00 | 23.00 | 23.00 | 23.00 | 23.00 | 23.00 | 23.00 |
| (A)/{(A)+(B)} (%) | | | 54.5 | 54.5 | 20.2 | 54.5 | 79.8 | 54.5 | 54.5 | 54.5 | 54.5 | 54.5 | 54.5 | 54.5 |
| {(A)+(B)}/{(A)+(B)+(C)} (%) | | | 94.0 | 94.0 | 94.0 | 94.0 | 94.0 | 94.0 | 94.0 | 94.0 | 94.0 | 94.0 | 94.0 | 94.0 |
| {(A)+(B)}/{(A)+(B)+(C)+(D)} (%) | | | 92.6 | 92.6 | 92.6 | 92.6 | 92.6 | 92.6 | 92.6 | 92.6 | 92.6 | 92.6 | 92.6 | 92.6 |
| {(D)}/{(A)+(B)+(C)+(D)} (%) | | | 1.5 | 1.5 | 1.5 | 1.5 | 1.5 | 1.5 | 1.5 | 1.5 | 1.5 | 1.5 | 1.5 | 1.5 |
| Evaluation results | Feeling in presence of sebum/in dry state | No stickiness | 14 | 9 | 7 | 7 | 8 | 14 | 15 | 12 | 12 | 13 | 13 | 11 |
| | | No stiffness | 13 | 12 | 15 | 15 | 12 | 12 | 12 | 12 | 12 | 12 | 13 | 14 |
| | | Good combability | 14 | 8 | 15 | 12 | 13 | 11 | 12 | 15 | 15 | 15 | 14 | 14 |
| | | Total score | 41 | 29 | 37 | 34 | 33 | 37 | 39 | 39 | 39 | 40 | 40 | 39 |
| | Shampoo resistance | $(\Delta E_2^* - \Delta E_1^*)$ | 0.6 | 11.3 | 1.4 | 0.8 | 0.5 | 2.1 | 1.5 | 10.0 | 0.8 | 0.7 | 1.7 | 0.5 |

[Table 3]

Table 3

| (mass%) | | | Example 1 | Example 25 |
|---|---|---|---|---|
| (A) | Trimethylsiloxysilicate | X-21-5595 *1 | 11.6 | 11.6 |
| (B) | (Acrylates/polytrimethylsiloxymethacrylate) copolymer | FA-4004ID *3 | 9.7 | 9.7 |
| (C) | High-molecular-weight dimethylpolysiloxane Viscosity 20,000,000 CS | Silsoft B3020 *4 | 1.35 | 1.35 |
| (D) | PEG/amodimethicone copolymer | SS-3588 *5 | 0.35 | 0.35 |
| (E) | Isododecane | Marukasol R *6 | Balance | Balance |
| (F) | Red 202 | | 1.5 | 0 |
| Total | | | 100 | 100 |
| (A) + (B) (mass%) | | | 21.30 | 21.30 |
| (A) + (B) + (C) (mass%) | | | 22.65 | 22.65 |
| (A) + (B) + (C) + (D) (mass%) | | | 23.00 | 23.00 |
| (A)/{(A)+(B)} (%) | | | 54.5 | 54.5 |
| {(A)+(B)}/{(A)+(B)+(C)} (%) | | | 94.0 | 94.0 |
| {(A)+(B)}/{(A)+(B)+(C)+(D)} (%) | | | 92.6 | 92.6 |
| {(D)}/{(A)+(B)+(C)+(D)} (%) | | | 1.5 | 1.5 |
| Evaluation results | Feeling in presence of sebum/in dry state | No stickiness | 14 | 14 |
| | | No stiffness | 13 | 13 |
| | | Combability | 14 | 14 |
| | | Total score | 41 | 41 |
| | Shampoo resistance | ($\Delta E_2$* - $\Delta E_1$*) | 0.6 | - |

The components described in the tables are as follows.

*1: X-21-5595, available from Shin-Etsu Chemical Co., Ltd., isododecane solution of trimethylsiloxysilicate (60 mass%)
*2: KP-550, available from Shin-Etsu Chemical Co., Ltd., isododecane solution of (acrylates/dimethicone) copolymer (40 mass%)
*3: DOWSIL FA 4004 ID Silicone Acrylate, available from Dow Toray Co., Ltd., isododecane solution of (acrylates/polytrimethylsiloxymethacrylate) copolymer (40 mass%)
*4: Silsoft B3020, available from Momentive Performance Materials Inc., high-molecular-weight dimethylpolysiloxane (20 mass%), viscosity at 25°C; 20,000,000 $mm^2/s$ (degree of polymerization: 3,546)
*5: DOWSII, SS-3588 Fluid, available from Dow Toray Co., Ltd., ethanol solution of (bisisobutyl PEG-15/amodimethicone) copolymer (80 mass%)
*6: Marukasol R, available from Maruzen Petrochemical Co., Ltd., isododecane
*7: DOWSIL FC-5002 IDD Resin Gum, isododecane solution of (trimethylsiloxysilicate/dimethiconol) crosspolymer (40 mass%)
*8: SilForm Flexible Resin, available from Momentive Performance Materials Inc., polymethylsilsesquioxane
*9: DOWSIL FA 4002 ID Silicone Acrylate, available from Dow Toray Co., Ltd., isododecane solution of (acrylates/polytrimethylsiloxymethacrylate) copolymer (40 mass%)
*10: KF-96H-100,000 CS, available from Shin-Etsu Chemical Co., Ltd., viscosity at 25°C; 100,000 $mm^2/s$ (degree of polymerization: 1,429)
*11: KF-96H-1,000,000 CS, available from Shin-Etsu Chemical Co., Ltd., high-molecular-weight dimethylpolysiloxane, viscosity at 25°C; 1,000,000 $mm^2/s$ (degree of polymerization: 2,233)
*12: X-25-9074, available from Shin-Etsu Chemical Co., Ltd., high-molecular-weight dimethylpolysiloxane, viscosity at 25°C; 30,000,000 $mm^2/s$ (degree of polymerization: 3,747)
*13: X-21-5666, available from Shin-Etsu Chemical Co., Ltd., cyclopentasiloxane solution of high-molecular-weight dimethiconol (30 mass%)
*14: KF-8018, available from Shin-Etsu Chemical Co., Ltd., cyclopentasiloxane solution of high-molecular-weight aminopropylmethylpolysiloxane (10 mass%)

Regarding the degree of polymerization (P) of the components *4 and 10 to 12, the molecular weight (M) can be determined from the viscosity ($\eta$) by Formula (4), and the degree of polymerization (P) can be determined by Formula (5) described below because the molecular weight of the basic unit of dimethylpolysiloxane is 74.

$$P = M/74 \qquad (5)$$

As is clear from Tables 1 to 3, the hair cosmetic composition of the present invention can impart a good feeling even when applied to hair in which sebum is present, and the film formed on the hair surface from the composition has also excellent washing durability.

Industrial Applicability

When the cosmetic composition of the present invention is applied to a target in which an oily component such as sebum is present, a good feeling can be imparted to the target, and a film having excellent washing durability can be formed.

## Claims

1. A cosmetic composition comprising components (A) to (D) described below:

   (A) a film-forming polymer including an M unit represented by $(R^1)_3SiO_{1/2}$ (wherein $R^1$ represents a hydrocarbon group having 1 or more and 12 or less carbons and being optionally substituted with fluorine, or a hydroxy group, and a plurality of $R^1$ may be the same or different), and a Q unit represented by $SiO_{4/2}$;
   (B) a film-forming polymer other than the component (A);
   (C) a high-molecular-weight organopolysiloxane; and
   (D) an organopolysiloxane other than the components (A) to (C), the organopolysiloxane having a polyalkylene oxide moiety and a cationic group.

2. The cosmetic composition according to claim 1, wherein the component (B) contains a silicone-based polymer.

3. The cosmetic composition according to claim 1 or 2, wherein the component (B) contains one or more selected from the group consisting of components (B1) and (B2) described below:

   (B 1) a silicone resin including a T unit represented by $R^1SiO_{3/2}$ and being substantially free of a Q unit represented by $SiO_{4/2}$ (wherein $R^1$ is as described above); and
   (B2) an acrylic silicone polymer.

4. The cosmetic composition according to claim 3, wherein the component (B2) contains an (acrylates/polytrimethylsiloxymethacrylate) copolymer.

5. The cosmetic composition according to any one of claims 1 to 4, wherein a degree of polymerization of the component (C) is 650 or more.

6. The cosmetic composition according to any one of claims 1 to 5, wherein a ratio [(A)/{(A) + (B)}] of a mass content of the component (A) to a total mass content of the component (A) and the component (B) in the composition is 10% or more and 95% or less.

7. The cosmetic composition according to any one of claims 1 to 6, wherein a total content of the components (A) to (D) in

the composition is 3.0 mass% or more.

8. The cosmetic composition according to any one of claims 1 to 7, wherein a ratio [{(A) + (B)}/{(A) + (B) + (C)}] of a total mass content of the component (A) and the component (B) to a total mass content of the components (A) to (C) in the composition is 70% or more.

9. The cosmetic composition according to any one of claims 1 to 8, wherein a ratio [(D)/{(A) + (B) + (C) + (D)}] of a mass content of the component (D) to a total mass content of the components (A) to (D) in the composition is 0.1% or more and 20% or less.

10. The cosmetic composition according to any one of claims 1 to 9, wherein the composition further comprises a volatile solvent as a component (E).

11. A method for treating a keratin substance, the method comprising applying the composition described in any one of claims 1 to 10 to a keratin substance and then drying the composition.

## INTERNATIONAL SEARCH REPORT

International application No.
**PCT/JP2023/034004**

**A. CLASSIFICATION OF SUBJECT MATTER**

***A61K 8/891***(2006.01)i; ***A61K 8/31***(2006.01)i; ***A61K 8/895***(2006.01)i; ***A61K 8/898***(2006.01)i; ***A61Q 5/00***(2006.01)i; ***A61Q 5/12***(2006.01)i
FI: A61K8/891; A61K8/31; A61K8/895; A61K8/898; A61Q5/00; A61Q5/12

According to International Patent Classification (IPC) or to both national classification and IPC

**B. FIELDS SEARCHED**

Minimum documentation searched (classification system followed by classification symbols)

A61K8/891; A61K8/31; A61K8/895; A61K8/898; A61Q5/00; A61Q5/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2021-187850 A (KAO CORP.) 13 December 2021 (2021-12-13) claims, paragraphs [0008], [0018], [0019], [0029]-[0032], [0047], [0048], [0058], [0084]-[0093] | 1-11 |
| Y | | 1-11 |
| Y | JP 7-196449 A (KOSE CORP.) 01 August 1995 (1995-08-01) claims, paragraphs [0001], [0004], [0032] | 1-11 |
| A | JP 2011-231073 A (DOW CORNING TORAY CO., LTD.) 17 November 2011 (2011-11-17) entire text | 1-11 |
| A | JP 4-045155 A (SHIN ETSU CHEM. CO., LTD.) 14 February 1992 (1992-02-14) entire text | 1-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 November 2023** | **28 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/034004**

| Patent document cited in search report | Publication date (day/month/year) | Patent family member(s) | Publication date (day/month/year) |
|---|---|---|---|
| JP 2021-187850 A | 13 December 2021 | US 2023/0210753 A1<br>claims, paragraphs [0044], [0062]-[0110], [0134], [0135], [0158], [0199]-[0212]<br>WO 2021/246272 A1<br>EP 4159190 A1<br>TW 202207899 A<br>CN 115666503 A | |
| JP 7-196449 A | 01 August 1995 | (Family: none) | |
| JP 2011-231073 A | 17 November 2011 | US 2013/0116340 A1<br>entire text<br>WO 2011/136389 A2<br>EP 2585033 A2<br>CN 103209679 A<br>KR 10-2013-0060198 A | |
| JP 4-045155 A | 14 February 1992 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016503433 T
- JP 2021187850 A
- JP H07196449 A
- JP H111530 A
- JP 2000063225 A
- JP H0225411 A
- JP H02132141 A
- JP H03162442 A
- JP 2003104825 A
- JP H0692825 A


### Non-patent literature cited in the description


- **NAKAMUTA**. *J. Chem, Soc. Japan*, 1956, 77588